(19) Europäisches Patentamt
European Patent Office
Office européen des brevets

(11) **EP 4 702 977 A1**

(12) **EUROPEAN PATENT APPLICATION**
published in accordance with Art. 153(4) EPC

(43) Date of publication:
**04.03.2026 Bulletin 2026/10**

(21) Application number: **24796108.9**

(22) Date of filing: **24.04.2024**

(51) International Patent Classification (IPC):
***A61K 31/365*** *(2006.01)* ***A61P 13/12*** *(2006.01)*
***C07D 307/93*** *(2006.01)* ***C07D 493/00*** *(2006.01)*

(52) Cooperative Patent Classification (CPC):
**A61K 31/365; A61K 31/4025; A61K 31/4178; A61K 31/443; A61K 31/4709; A61K 31/506; A61P 13/12; A61P 19/06; C07D 307/93; C07D 405/12; C07D 407/12; C07D 493/00**

(86) International application number:
**PCT/CN2024/089520**

(87) International publication number:
**WO 2024/222731 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.04.2023 CN 202310467233**

(71) Applicant: **Hangzhou E & J Biopharmaceutical Technology Co., Ltd.**
**Hangzhou, Zhejiang 311112 (CN)**

(72) Inventor: **XU, Yuanjian**
**Hangzhou, Zhejiang 311112 (CN)**

(74) Representative: **Gille Hrabal Partnerschaftsgesellschaft mbB**
**Patentanwälte**
**Brucknerstraße 20**
**40593 Düsseldorf (DE)**

(54) **URIC ACID TRANSPORTER AGONIST COMPOUND, PHARMACEUTICAL COMPOSITION, AND PREPARATION METHOD THEREFOR AND USE THEREOF**

(57) A uric acid transporter agonist compound as represented by formula (I), a pharmaceutical composition, and a preparation method therefor and the use thereof. The compound as a drug is hardly absorbed into an in-vivo circulation system, and has good uric acid transport protein agonistic activity.

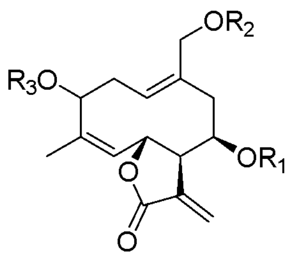

(I)

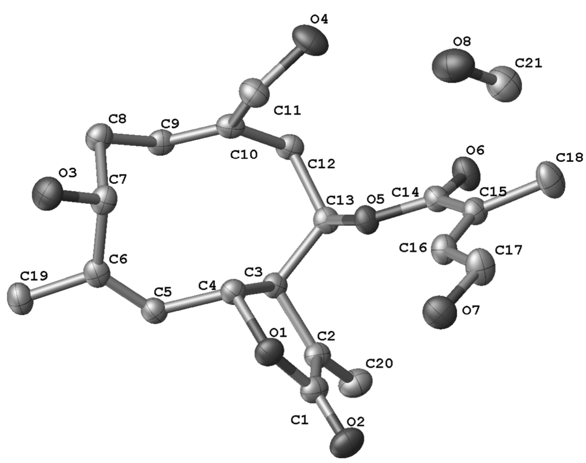

FIG. 1

EP 4 702 977 A1

## Description

The present application claims priority to the prior application with the patent application No. 2023104672331 filed to the China National Intellectual Property Administration by the applicant on April 25, 2023 and entitled "URIC ACID TRANSPORTER AGONIST COMPOUND, PHARMACEUTICAL COMPOSITION, AND PREPARATION METHOD THEREFOR AND USE THEREOF", which is incorporated herein by reference in its entirety.

## TECHNICAL FIELD

The present disclosure pertains to the field of pharmaceuticals, and particularly relates to a uric acid transporter agonist compound, a pharmaceutical composition, a preparation method therefor, and use thereof.

## BACKGROUND

Uric acid is the final product of purine metabolism in humans and apes, and is excreted primarily through pathways such as the kidneys, intestines, and the like. Increased uric acid production and impaired uric acid excretion in the human body will lead to an increase in the uric acid concentration in the blood, i.e., hyperuricemia (HUA). According to the "China Multi-Disciplinary Expert Consensus on Diagnosis and Treatment of Hyperuricemia and Related Diseases" issued by the Chinese Medical Association in 2017, HUA is defined as a fasting serum uric acid level of >420 μmol/L (7 mg/dL) for males and >360 μmol/L (6 mg/dL) for females on two different days under a normal purine diet. The limit is based on the fact that the saturated concentration of urate in the blood is 420 μmol/L. Above this value, urate will deposit in synovial membranes of joints, bursae, cartilage, and other tissues, thereby causing gout and even leading to serious consequences such as joint injury and kidney function impairment.

Foods such as seafood, meat products, beer, and the like are rich in purine compounds, so gout is also called "the arthritis of the wealthy" in the era of material scarcity. However, with the significant improvement in the overall living standard in China in recent years, the number of hyperuricemia patients has surged, making it the second most prevalent metabolic disease after diabetes.

Although uric acid excretion primarily occurs through the kidneys, the intestines also serve as an important organ for uric acid excretion. Only about one-third of the uric acid excretion in humans is excreted through the intestines, but for patients with kidney function impairment, the intestines can become the main channel for the excretion of uric acid. Intestinal cells express a large number of transporters having uric acid transport functions, among which adenosine triphosphate binding cassette transporter G2 (ABCG2) is mainly expressed in intestinal and renal epithelial cells and is a transporter capable of unidirectional excretion of uric acid. A decrease in the uric acid excretion function of ABCG2 can cause hyperuricemia. Additionally, MRP2 (multidrug resistance protein), MRP4, GLUT9 (glucose transport 9), OAT10 (organic anion transporter), and the like can all transport uric acid and are expressed in intestinal cells. Activating and enhancing transporters having the uric acid transport and excretion function in the intestines can effectively lower the uric acid concentration in the blood. The compound involved in the present disclosure can activate and enhance the uric acid transport function in intestinal epithelial cells in the intestines, so that uric acid in the blood can be excreted into the digestive tract, thereby lowering the uric acid concentration in the blood. Further, the amount of uric acid that needs to be excreted through the kidneys can be reduced, thereby reducing the burden on the kidneys and improving the kidney function.

At present, uric acid-reducing drugs mainly fall into two categories. One is represented by allopurinol and febuxostat, which reduce uric acid synthesis by inhibiting xanthine oxidase activity, and the other is represented by benzbromarone, which inhibits uric acid reabsorption in renal tubules by inhibiting URAT1 (renal tubular uric acid transporter 1) to promote uric acid excretion. Although the two categories of drugs have relatively good uric acid-reducing effects, they cause certain damage to liver and kidney functions, and hyperuricemia patients may suffer from kidney function diseases. Therefore, there is an urgent need to develop novel uric acid-reducing drugs with low toxic and side effects.

## SUMMARY

To solve the technical problems described above, the present disclosure provides a compound represented by formula (I) and a racemate, a stereoisomer, a tautomer, an isotopically labeled compound, a nitrogen oxide, a solvate, a polymorph, a metabolite, an ester, a prodrug, or a pharmaceutically acceptable salt thereof:

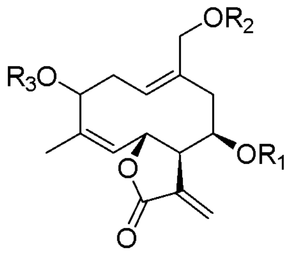

(I)

wherein

$R_1$ is selected from H, deuterium, and the following groups that are unsubstituted or optionally substituted with one, two, or more $R_a$: $C_{1-12}$ alkyl, $C_{3-12}$ cycloalkyl, 3- to 14-membered heterocyclyl, $C_{6-14}$ aryl, 5- to 14-membered heteroaryl, and -C(O)-$R_{11}$;
$R_2$ is selected from H, deuterium, and -C(O)-$R_{21}$ that is unsubstituted or optionally substituted with one, two, or more $R_b$;
$R_3$ is selected from H, deuterium, and $C_{1-12}$ alkyl, $C_{6-14}$ aryl, and 5- to 14-membered heteroaryl that are unsubstituted or optionally substituted with one, two, or more $R_c$;
$R_{11}$ and $R_{21}$ are identical or different and are independently selected from $C_{1-12}$ alkyl, $C_{2-12}$ alkenyl, $C_{2-12}$ alkynyl, $C_{3-12}$ cycloalkyl, 3- to 14-membered heterocyclyl, $C_{6-14}$ aryl, and 5- to 14-membered heteroaryl;
each of $R_a$, $R_b$, and $R_c$ is identical or different and is independently selected from H, deuterium, halogen, CN, OH, $C_{1-12}$ alkyl, and $C_{1-12}$ alkyloxy.

According to an embodiment of the present disclosure, $R_1$ is selected from H and $C_{1-6}$ alkyl, $C_{3-8}$ cycloalkyl, 3- to 8-membered heterocyclyl, $C_{6-10}$ aryl, 5- to 10-membered heteroaryl, and -C(O)-$R_{11}$; that are unsubstituted or optionally substituted with one, two, or more $R_a$.

According to an embodiment of the present disclosure, $R_1$ is selected from H, methyl, ethyl, isopropyl, cyclopropyl, tetrahydropyranyl, piperidyl, phenyl, pyridinyl, pyrimidinyl, methylpyrrolyl, methylimidazolyl, thienyl, naphthyl, quinolyl, methylpyridinyl, tolyl, fluorophenyl, and

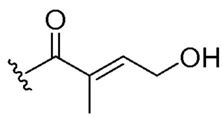.

According to an embodiment of the present disclosure, $R_1$ is selected from H, methyl, ethyl, isopropyl, cyclopropyl, phenyl,

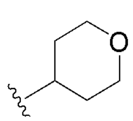, 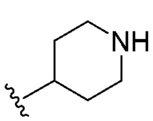, 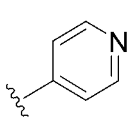, 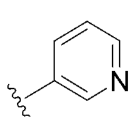, 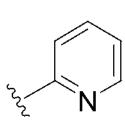, 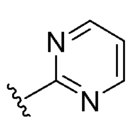, 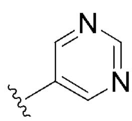, 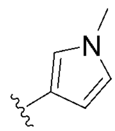,

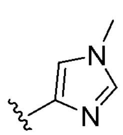, 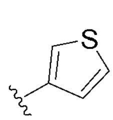, 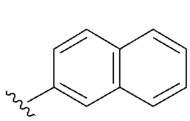, 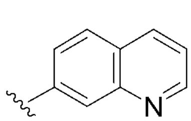, 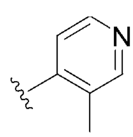, 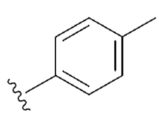, 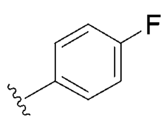,

and

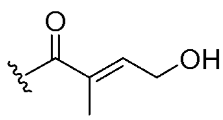.

According to an embodiment of the present disclosure, $R_2$ is selected from H and -C(O)-$R_{21}$.

According to an embodiment of the present disclosure, $R_2$ is H.

According to an embodiment of the present disclosure, $R_3$ is selected from H, deuterium, and $C_{1-6}$ alkyl and $C_{6-10}$ aryl that are unsubstituted or optionally substituted with one, two, or more $R_c$.

According to an embodiment of the present disclosure, $R_{11}$ is selected from the following groups that are unsubstituted or optionally substituted with one, two, or more $R_a$: $C_{1-6}$ alkyl, $C_{2-6}$ alkenyl, $C_{2-6}$ alkynyl, $C_{6-10}$ aryl, and 5- to 10-membered heteroaryl.

According to an embodiment of the present disclosure, $R_{11}$ is selected from

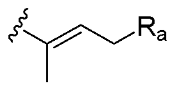
.

According to an embodiment of the present disclosure, $R_{21}$ is selected from $C_{1-6}$ alkyl, $C_{2-6}$ alkenyl, $C_{2-6}$ alkynyl, $C_{6-10}$ aryl, and 5- to 10-membered heteroaryl.

According to an embodiment of the present disclosure, each of $R_a$, $R_b$, and $R_c$ is identical or different and is independently selected from H, OH, F, and $C_{1-6}$ alkyl (e.g., methyl).

According to an embodiment of the present disclosure, the compound of formula (I) has a structure represented by formula (I'):

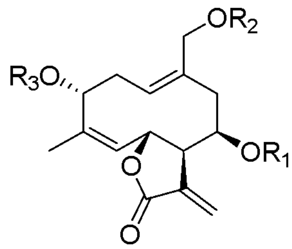
(I'),

wherein $R_1$, $R_2$, and $R_3$ are independently defined as described above.

According to an embodiment of the present disclosure, the compound of formula (I) has a structure represented by formula (I-1) or (I-2):

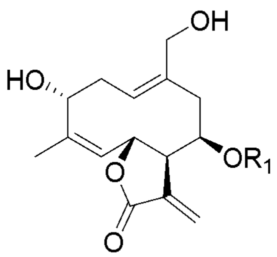
(I-1)

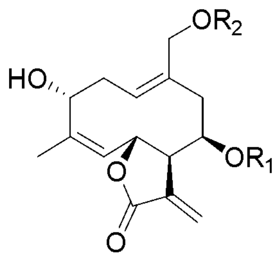
(I-2),

wherein $R_1$ and $R_2$ are independently defined as described above.

According to an embodiment of the present disclosure, the compound of formula (I) has a structure represented by formula (II):

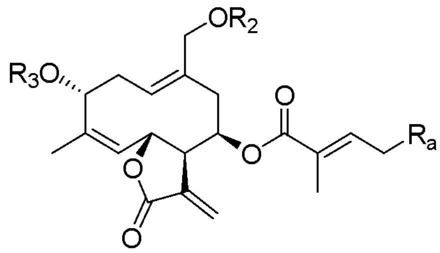

(II)

wherein $R_2$, $R_3$, and $R_a$ are independently defined as described above.

According to an embodiment of the present disclosure, the compound of formula (I) has a structure represented by formula (II-1):

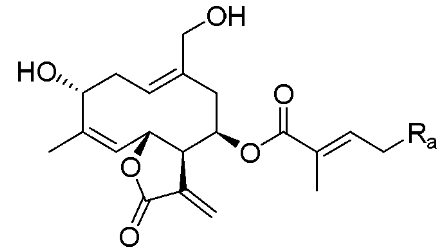

(II-1)

wherein $R_a$ is defined as described above.

According to an embodiment of the present disclosure, the compound of formula (I) is selected from the following structures:

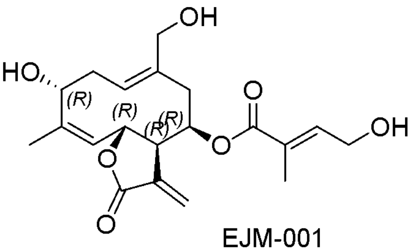
EJM-001 ,

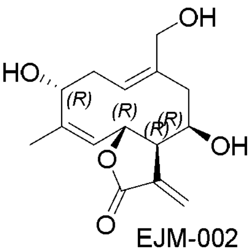
EJM-002 ,

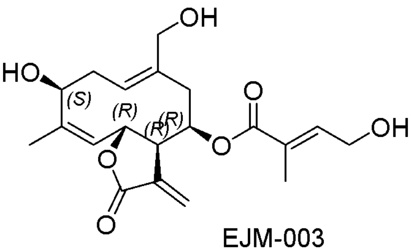
EJM-003 ,

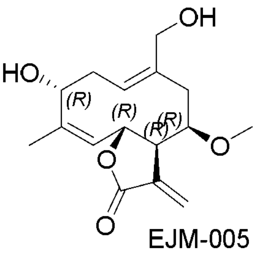
EJM-005 ,

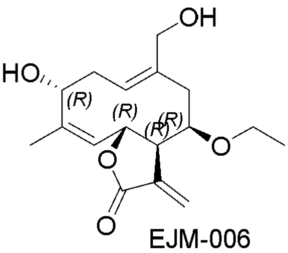
EJM-006 ,

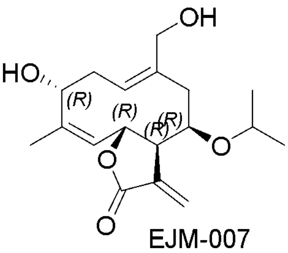
EJM-007 ,

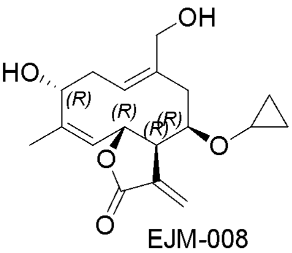
EJM-008 ,

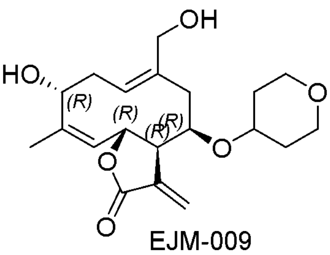
EJM-009 ,

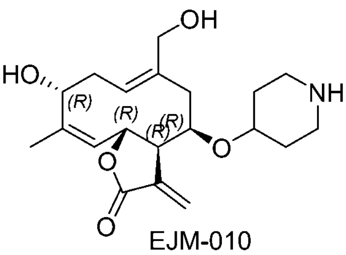
EJM-010 ,

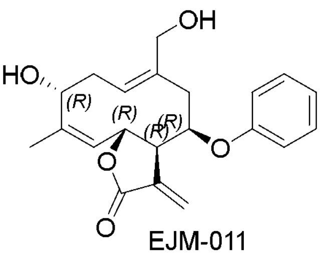
EJM-011 ,

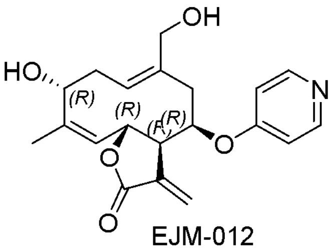
EJM-012 ,

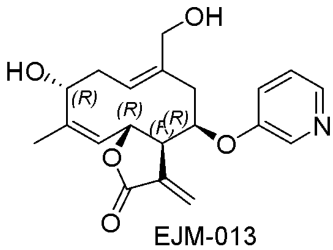
EJM-013 ,

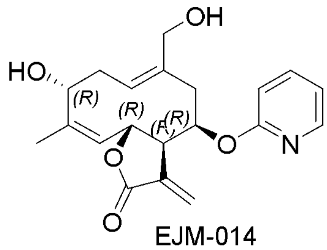
EJM-014 ,

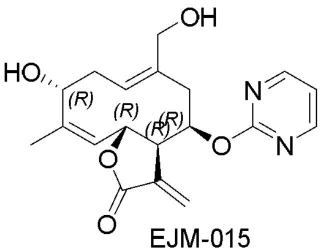
EJM-015 ,

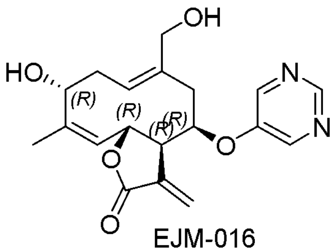
EJM-016 ,

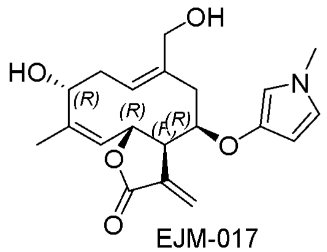
EJM-017 ,

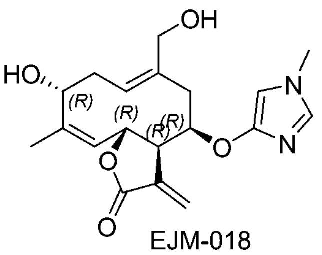
EJM-018 ,

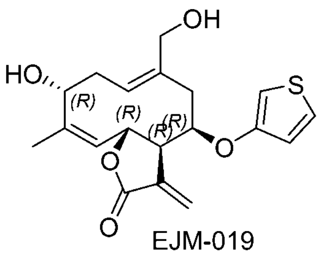
EJM-019 ,

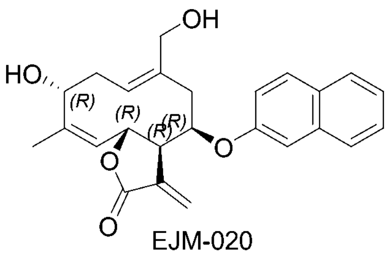
EJM-020 ,

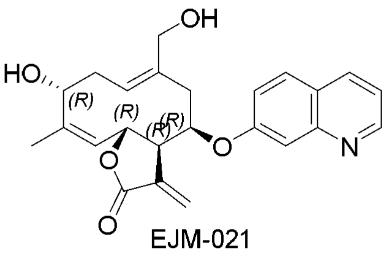
EJM-021 ,

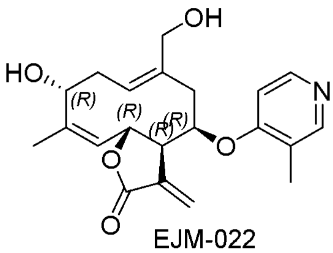
EJM-022 ,

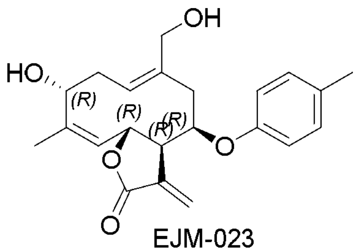
EJM-023 ,

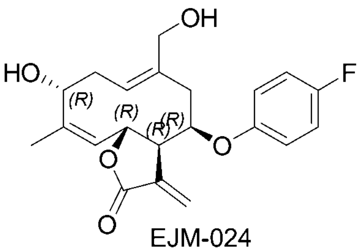
EJM-024 ,

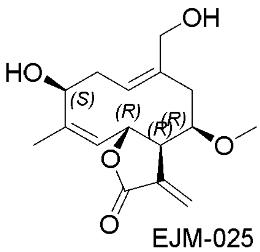
EJM-025 ,

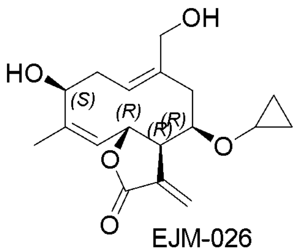
EJM-026 ,

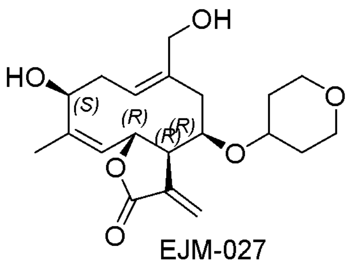
EJM-027 ,

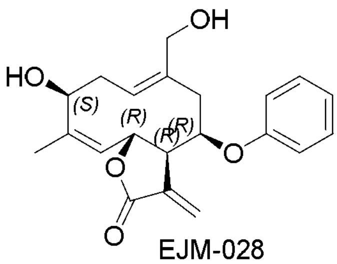
EJM-028 ,

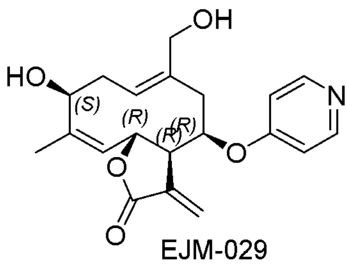
EJM-029 ,

and

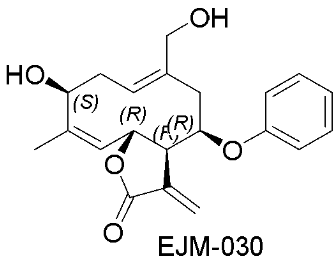

EJM-030

.

The present disclosure also provides a pharmaceutical composition comprising the compound of formula (I) and the racemate, the stereoisomer, the tautomer, the isotopically labeled compound, the nitrogen oxide, the solvate, the polymorph, the metabolite, the ester, the prodrug, or the pharmaceutically acceptable salt thereof described in the present disclosure.

In some embodiments, the pharmaceutical composition described in the present disclosure further comprises a therapeutically effective amount of the compound of formula I and the racemate, the stereoisomer, the tautomer, the isotopically labeled compound, the nitrogen oxide, the solvate, the polymorph, the metabolite, the ester, the prodrug, or the pharmaceutically acceptable salt thereof described in the present disclosure, and a pharmaceutically acceptable carrier.

The carrier in the pharmaceutical composition is "acceptable" in that it is compatible with (and preferably, capable of stabilizing) the active ingredient of the composition and is not deleterious to the subject being treated. One or more pharmaceutical excipients can be used for delivery of the active compound.

The present disclosure further provides use of the compound of formula I and the racemate, the stereoisomer, the tautomer, the isotopically labeled compound, the nitrogen oxide, the solvate, the polymorph, the metabolite, the ester, the prodrug, or the pharmaceutically acceptable salt thereof, or the pharmaceutical composition for the manufacturing of a medicament.

According to some embodiments, the medicament is a medicament for diagnosing, preventing, and/or treating a disease or condition caused by abnormal uric acid levels.

According to some embodiments, the medicament is a uric acid transporter agonist.

According to some embodiments, the disease or condition is selected from hyperuricemia and gout.

The present disclosure also provides a method for diagnosing, preventing, and/or treating a disease or condition caused by abnormal uric acid levels, and the method comprises administering to a patient in need of such treatment a therapeutically effective amount of at least one compound of the present disclosure alone, or optionally, in combination with another compound of the present disclosure and/or at least one additional type of therapeutic agent. According to the present disclosure, the disease or condition is selected from hyperuricemia and gout.

The compounds of the present disclosure may be used in combination with an additional therapeutic agent.

**Beneficial Effects**

The compounds provided by the present disclosure can improve the expression of ABCG2, have good uric acid transporter agonistic activity, and can activate and enhance the uric acid transport capacity of the intestines, thereby reducing the blood uric acid concentration. The compounds prepared in the present disclosure are hardly absorbed into the *in vivo* circulatory system, and thus the toxic and side effects that the compounds may have on other organs in the body are greatly reduced. Moreover, the half maximal inhibitory concentration of the compounds for intestinal cells (Caco2 cell test) exceeds 400 μmol/L, and the toxicity is extremely low. Animal tests have also shown that such compounds have little toxicity.

In addition, the compounds of the present disclosure reduce the burden on the liver and kidneys by reducing the blood uric acid concentration, thereby improving the function of the liver and kidneys, and further reducing the concentration of liver transaminase and serum creatinine.

**BRIEF DESCRIPTION OF THE DRAWINGS**

FIG. 1 is a diagram showing the crystal structure of compound EJM-001.
FIG. 2 is a $^1H$ NMR ($CDCl_3$) spectrum of compound EJM-001.
FIG. 3 is a diagram showing the crystal structure of compound EJM-002.

FIG. 4 is a $^1H$ NMR ($CDCl_3$) spectrum of compound EJM-002.
FIG. 5 is a diagram showing the crystal structure of compound EJM-003.
FIG. 6 is a $^1H$ NMR ($CDCl_3$) spectrum of compound EJM-003.
FIG. 7 shows a fitted curve of intestinal absorption of compound EJM-001 in rats.
FIG. 8 shows a fitted curve of intestinal absorption of compound EJM-002 in rats.
FIG. 9 shows a comparison of the blood uric acid concentration tests in hyperuricemia model mice treated with the compounds of the present disclosure.
FIG. 10 shows changes in ABCG2 expression in Caco2 cells after incubation with EJM001 at different concentrations for 12/24/48 h.

($ indicates a significant difference from DMSO-12 h, $P < 0.05$; $$ indicates a significant difference from DMSO-12 h, $P < 0.01$; $$$ indicates a significant difference from DMSO-12 h, $P < 0.001$;
* indicates a significant difference from DMSO-24 h, $P < 0.05$; ** indicates a significant difference from DMSO-24 h, $P < 0.01$; *** indicates a significant difference from DMSO-24 h, $P < 0.001$;
# indicates a significant difference from DMSO-48 h, $P < 0.05$; ## indicates a significant difference from DMSO-48 h, $P < 0.01$; ### indicates a significant difference from DMSO-48 h, $P < 0.001$.)

**Definitions and Description**

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should be construed as being within the scope of the specification and/or the claims of the present application.

The term "optional" (or "optionally") in the definition of the general formula of the present application means a situation where a group is substituted with zero, one, or more substituents. For example, "optionally substituted with one, two, or more R" means that the group may not be substituted with R (unsubstituted) or may be optionally substituted with one, two, or more R.

"More" refers to three or more.

Unless otherwise stated, a numerical range set forth in the specification and claims shall be construed as at least including each specific integer value within the range. For example, the numerical range of "1-14" shall be construed as including each integer value in the numerical range of "1-14", i.e., 1, 2, 3, 4, 5, 6, 7, 8, 9, 10, 11, 12, 13, and 14. The term "$C_{1-12}$ alkyl" should be understood to refer to linear or branched alkyl groups having 1-12 carbon atoms, "$C_{1-8}$ alkyl" refers to linear or branched alkyl groups having 1, 2, 3, 4, 5, 6, 7, or 8 carbon atoms, and "$C_{1-6}$ alkyl" refers to linear or branched alkyl groups having 1, 2, 3, 4, 5, or 6 carbon atoms. The alkyl is, for example, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, or the like, or an isomer thereof.

The term "$C_{2-12}$ alkenyl" should be understood to refer to a linear or branched monovalent hydrocarbyl group containing one or more double bonds and having 2-12 carbon atoms, preferably "$C_{2-10}$ alkenyl". "$C_{2-10}$ alkenyl" should be understood to preferably refer to a linear or branched monovalent hydrocarbyl group containing one or more double bonds and having 2, 3, 4, 5, 6, 7, 8, 9, or 10 carbon atoms, more preferably "$C_{2-8}$ alkenyl". "$C_{2-10}$ alkenyl" should be understood to preferably refer to a linear or branched monovalent hydrocarbyl group containing one or more double bonds and having 2, 3, 4, 5, 6, 7, or 8 carbon atoms, for example, having 2, 3, 4, 5, or 6 carbon atoms (i.e., $C_{2-6}$ alkenyl) or having 2 or 3 carbon atoms (i.e., $C_{2-3}$ alkenyl). It should be understood that in the case that the alkenyl contains more than one double bond, the double bonds can be separated from one another or conjugated. The alkenyl is, for example, ethenyl, allyl, (E)-2-methylethenyl, (Z)-2-methylethenyl, (E)-but-2-enyl, (Z)-but-2-enyl, (E)-but-1-enyl, (Z)-but-1-enyl, pent-4-enyl, (E)-pent-3-enyl, (Z)-pent-3-enyl, (E)-pent-2-enyl, (Z)-pent-2-enyl, (E)-pent-1-enyl, (Z)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methylbut-2-enyl, (*E*)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propylvinyl, or 1-isopropylvinyl.

The term "$C_{2-12}$ alkynyl" should be understood to refer to a linear or branched monovalent hydrocarbyl group containing one or more triple bonds and having 2-12 carbon atoms, preferably "$C_{2-10}$ alkynyl". The term "$C_{2-10}$ alkynyl"

should be understood to preferably refer to a linear or branched monovalent hydrocarbyl group containing one or more triple bonds and having 2, 3, 4, 5, 6, 7, 8, 9, or 10 carbon atoms, for example, having 2, 3, 4, 5, 6, 7, or 8 carbon atoms (i.e., "$C_{2-8}$ alkynyl"), having 2, 3, 4, 5, or 6 carbon atoms (i.e., "$C_{2-6}$ alkynyl"), or having 2 or 3 carbon atoms ("$C_{2-3}$ alkynyl"). The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl. In particular, the alkynyl is ethynyl, prop-1-ynyl, or prop-2-ynyl.

The term "$C_{3-12}$ cycloalkyl" should be understood to refer to a saturated monovalent monocyclic or bicyclic (e.g., fused, bridged, or spiro) hydrocarbon ring or tricyclic alkane having 3-12 carbon atoms, preferably "$C_{3-10}$ cycloalkyl", and more preferably "$C_{3-8}$ cycloalkyl". The term "$C_{3-12}$ cycloalkyl" should be understood to refer to a saturated monovalent monocyclic or bicyclic (e.g., bridged or spiro) hydrocarbon ring or tricyclic alkane having 3, 4, 5, 6, 7, 8, 9, 10, 11, or 12 carbon atoms. The $C_{3-12}$ cycloalkyl may be monocyclic hydrocarbyl such as cyclopropyl, cyclobutyl, cyclopentyl, cyclohexyl, cycloheptyl, cyclooctyl, cyclononyl or cyclodecyl, or bicyclic hydrocarbyl such as bornyl, indolyl, hexahydroindolyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, 2,7-diazaspiro[3,5]nonyl, 2,6-diazaspiro[3,4]octyl, or tricyclic hydrocarbyl such as adamantyl. The term "$C_{6-14}$ aryl" should be understood to preferably refer to an aromatic or partially aromatic monovalent monocyclic, bicyclic (e.g., fused, bridged, or spiro), or tricyclic hydrocarbon ring having 6-14 carbon atoms, which may be a single aromatic ring or multiple aromatic rings fused together, preferably "$C_{6-10}$ aryl". The term "$C_{6-14}$ aryl" should be understood to preferably refer to an aromatic or partially aromatic monovalent monocyclic, bicyclic, or tricyclic hydrocarbon ring having 6, 7, 8, 9, 10, 11, 12, 13, or 14 carbon atoms ("$C_{6-14}$ aryl"), in particular a ring having 6 carbon atoms ("$C_6$ aryl"), e.g., phenyl; biphenyl; a ring having 9 carbon atoms ("$C_9$ aryl"), e.g., indanyl or indenyl; a ring having 10 carbon atoms ("$C_{10}$ aryl"), e.g., tetrahydronaphthyl, dihydronaphthyl, or naphthyl; a ring having 13 carbon atoms ("$C_{13}$ aryl"), e.g., fluorenyl; or a ring having 14 carbon atoms ("$C_{14}$ aryl"), e.g., anthryl. When the $C_{6-20}$ aryl is substituted, it may be monosubstituted or polysubstituted. In addition, the substitution site is not limited, and the substitution may be, for example, ortho-substitution, para-substitution, or meta-substitution.

The term "5- to 14-membered heteroaryl" should be understood to refer to a monovalent monocyclic, bicyclic (e.g., fused, bridged, or spiro), or tricyclic aromatic ring system, which has 5-14 ring atoms and contains 1-5 heteroatoms independently selected from N, O, and S, such as "5- to 10-membered heteroaryl". The term "5- to 14-membered heteroaryl" should be understood to refer to a monovalent monocyclic, bicyclic, or tricyclic aromatic ring system, which has 5, 6, 7, 8, 9, 10, 11, 12, 13, or 14 ring atoms, in particular 5, 6, 9, or 10 carbon atoms, contains 1-5, preferably 1-3 heteroatoms independently selected from N, O, and S, and may be benzo-fused in each case.

Unless otherwise defined, the term "3- to 14-membered heterocyclyl" refers to a saturated or unsaturated non-aromatic ring or ring system; for example, it is a 4-, 5-, 6-, or 7-membered monocyclic ring system, a 7-, 8-, 9-, 10-, 11-, or 12-membered bicyclic (e.g., fused, bridged, or spiro) ring system, or a 10-, 11-, 12-, 13-, or 14-membered tricyclic ring system, and contains at least one, e.g., 1, 2, 3, 4, 5, or more heteroatoms selected from O, S, and N, wherein N and S may also be optionally oxidized to various oxidized forms to form nitrogen oxides, -S(O)-, or - $S(O)_2$-. For example, the "3- to 14-membered heterocyclyl" may be 3- to 14-membered N-containing heterocyclyl (containing at least one N). Preferably, the heterocyclyl may be selected from "3- to 10-membered heterocyclyl". The term "3- to 10-membered heterocyclyl" refers to a saturated or unsaturated non-aromatic ring or ring system and contains at least one heteroatom selected from O, S, and N. The heterocyclyl may be attached to the remainder of the molecule via any one of the carbon atoms or a nitrogen atom (if present). The heterocyclyl may include fused or bridged rings as well as spiro rings. In particular, the heterocyclyl may include, but is not limited to, 4-membered rings such as azetidinyl and oxetanyl; 5-membered rings such as tetrahydrofuranyl, dioxolyl, pyrrolidinyl, imidazolidinyl, pyrazolidinyl, and pyrrolinyl; or 6-membered rings such as tetrahydropyranyl, piperidyl, morpholinyl, dithianyl, thiomorpholinyl, piperazinyl, and trithianyl; or 7-membered rings such as diazepanyl. Optionally, the heterocyclyl may be benzo-fused. The heterocyclyl may be bicyclic, for example, but is not limited to, a 5,5-membered ring such as a hexahydrocyclopenta[c]pyrrol-2(1H)-yl ring, or a 5,6-membered bicyclic ring such as a hexahydropyrrolo[1,2-*a*]pyrazin-2(1*H*)-ylring. The heterocyclyl may be partially unsaturated, i.e., it may contain one or more double bonds, for example, but is not limited to, dihydrofuranyl, dihydropyranyl, 2,5-dihydro-1H-pyrrolyl, 4H-[1,3,4]thiadiazinyl, 1,2,3,5-tetrahydrooxazolyl, or 4H-[1,4]thiazinyl, or it may be benzo-fused, for example, but is not limited to, dihydroisoquinolyl. When the 3- to 14-membered heterocyclyl is connected to another group to form the compound of the present disclosure, the group may be connected to the carbon atom on the 3- to 14-membered heterocyclyl, or may be connected to the heteroatom on the 3- to 14-membered heterocyclyl. For example, when the 3- to 14-membered heterocyclyl is selected from piperazinyl, the group may be connected to the nitrogen atom on the piperazinyl. Alternatively, when the 3- to 14-membered heterocyclyl is selected from piperidyl, the group may be connected to the nitrogen atom or the carbon atom in the para position on the piperidyl ring.

The term "halogen" refers to fluorine, chlorine, bromine, and iodine.

"Halo" refers to substitution with one or more halogens.

It will be appreciated by those skilled in the art that the compound represented by formula (I) may be present in the form of various pharmaceutically acceptable salts. If these compounds have basic centers, they can form acid addition salts; if these compounds have acidic centers, they can form base addition salts; if these compounds comprise both acidic centers (e.g., carboxyl) and basic centers (e.g., amino), they can also form internal salts.

The compounds of the present disclosure may be present in the form of a solvate (e.g., hydrate), and the compounds of the present disclosure contain a polar solvent as a structural element of the crystal lattice of the compound, particularly, for example, water, methanol, or ethanol. The amount of the polar solvent, especially water, may be present in a stoichiometric or non-stoichiometric ratio.

According to the molecular structure, the compounds of the present disclosure may be chiral and may therefore be present in various enantiomeric forms. These compounds may therefore be present in a racemic or optically active form. The compounds of the present disclosure encompass isomers with each chiral carbon in R or S configuration, or mixtures and racemates thereof. The compounds of the present disclosure or intermediates thereof may be separated into enantiomers by chemical or physical methods well known to those skilled in the art, or used in such form for synthesis. In the case of racemic amines, diastereoisomers are prepared from mixtures by reaction with optically active resolving agents. Examples of suitable resolving agents are optically active acids such as *R*- or *S*-tartaric acid, diacetyltartaric acid, dibenzoyltartaric acid, mandelic acid, malic acid, lactic acid, suitable *N*-protected amino acids (e.g., *N*-benzoylproline or *N*-benzenesulfonylproline), or various optically active camphorsulfonic acids. Enantiomeric resolution by chromatography can be advantageously conducted with the aid of optically active resolving agents, such as dinitrobenzoylphenylglycine, cellulose triacetate or other carbohydrate derivatives or chirally derivatized methacrylate polymers immobilized on silica gel. Suitable eluents for this purpose are mixtures of solvents containing water or alcohol, for example, hexane/isopropanol/acetonitrile.

The corresponding stable isomers can be separated according to known methods, such as extraction, filtration, or column chromatography.

The term "patient" refers to any animal including mammals, preferably mice, rats, other rodents, rabbits, dogs, cats, pigs, cattle, sheep, horses, or primates, and most preferably humans.

The term "therapeutically effective amount" refers to the amount of the active compound or drug that causes a biological or medical response that researchers, veterinarians, physicians, or other clinicians are looking for in tissues, systems, animals, individuals, or humans, including one or more of the following effects: (1) disease prevention: for example, the prevention of a disease, disorder, or condition in an individual who is susceptible to the disease, disorder, or condition but has not yet experienced or exhibited the pathology or symptoms of the disease; (2) disease inhibition: for example, the inhibition of a disease, disorder, or condition in an individual who is experiencing or exhibiting the pathology or symptoms of the disease, disorder, or condition (i.e., the prevention of the further development of the pathology and/or symptoms); and (3) disease alleviation: for example, the alleviation of a disease, disorder, or condition in an individual who is experiencing or exhibiting the pathology or symptoms of the disease, disorder, or condition (i.e., the reverse of the pathology and/or symptoms).

## DETAILED DESCRIPTION

The technical solutions of the present disclosure will be further illustrated in detail with reference to the following specific examples. It should be understood that the following examples are merely exemplary illustrations and explanations of the present disclosure, and should not be construed as limiting the protection scope of the present disclosure. All techniques implemented based on the content of the present disclosure described above are encompassed within the protection scope of the present disclosure.

Unless otherwise stated, the raw materials and reagents used in the following examples are all commercially available products or can be prepared by using known methods.

### Example 1. Preparation of EJM-001:

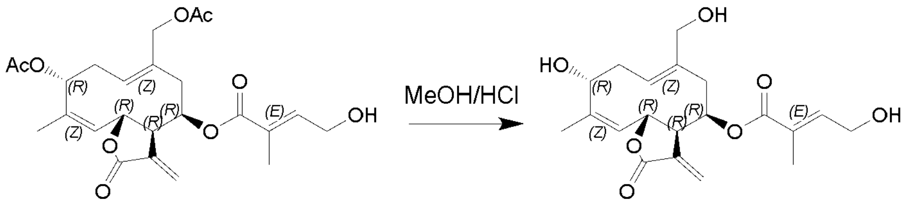


Eupalinolide I EJM-001

Eupalinolide I was dissolved in 6 volumes of methanol. The mixture was cooled to 0-5 °C, and 0.65 volumes of concentrated hydrochloric acid were added. The mixture was reacted for 48 h. After the reaction was completed, the pH was adjusted to about 7 with sodium hydroxide, and the solvent was removed by rotary evaporation. The organic substance was then dissolved in methanol, and the mixture was filtered to remove the inorganic salt. Finally, the product was recrystallized from methanol to give pure EJM-001 with HPLC purity of >98% (methanol in the crystal was subtracted). FIG. 1 and FIG. 2 are the crystal structure and nuclear magnetic resonance spectrum of EJM-001, respectively.

**Example 2. Preparation of EJM-002:**

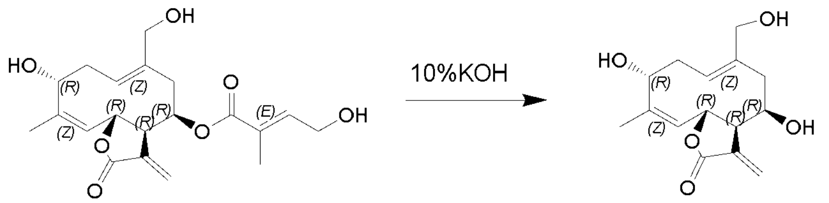


EJM-001 EJM-002

Method 1: EJM-001 was dissolved in 6 volumes of a 10% KOH solution, and the mixture was stirred at room temperature for 10 min. The pH was adjusted to about 7 with hydrochloric acid, and the mixture was extracted with ethyl acetate and concentrated. The product was then crystallized from ethyl acetate to give pure EJM-002 with HPLC purity of >98%.

Method 2: EJM-001 was dissolved in 6 volumes of methanol, and potassium carbonate in an amount of 0.75 times the weight of EJM-001 was added. The mixture was stirred at room temperature for 5 h and filtered, and the solvent was then removed by rotary evaporation. The residue was dissolved in ethyl acetate, and the mixture was concentrated. The product was crystallized from ethyl acetate to give pure EJM-002 with HPLC purity of >98%.

FIG. 3 and FIG. 4 are the crystal structure and nuclear magnetic resonance spectrum of EJM-002, respectively.

**Example 3. Preparation of EJM-003:**

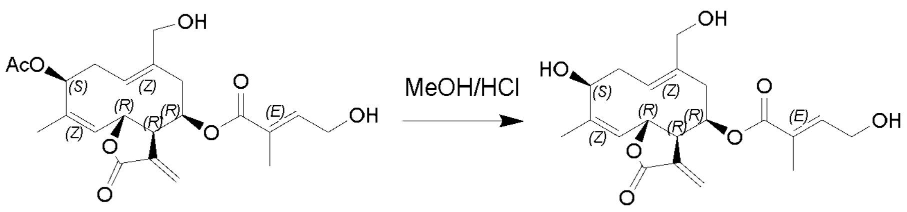


EJM-004
Eupalinolide F

EJM-003

Eupalinolide F was dissolved in 6 volumes of methanol. The mixture was cooled to 0-5 °C, and 0.65 volumes of concentrated hydrochloric acid were added. The mixture was reacted for 48 h. After the reaction was completed, the pH was adjusted to about 7 with sodium hydroxide, and the solvent was removed by rotary evaporation. The organic substance was then dissolved in methanol, and the mixture was filtered to remove the inorganic salt. Finally, the product

was recrystallized from ethyl acetate to give pure EJM-003 with HPLC purity of >98%. FIG. 5 and FIG. 6 are the crystal structure and nuclear magnetic resonance spectrum of EJM-003, respectively.

**Test Example 1. Intestinal Absorption Test in Rats**

**1. Experimental objective: to study the intestinal absorption of EJM-001 and EJM-002 in rats**
**2. Experimental reagents and materials:**
**Control solution**

**2.1. K-R test solution (pH 7.4)**
($CaCl_2$, glucose, NaCl, KCl, $NaHCO_3$, $NaH_2PO_4$, $MgCl_2$)
**2.2. Blank intestinal perfusion solution**
**2.3. Intestinal perfusion solution (0.3 mg/mL)**
**2.4. 20%** urethane solution (anesthesia): 1.5 g/200 g

**3. Experimental animals:**
Male SD rats (220±20 g, SPF-grade)
**4. Preparation of solutions**

**4.1. Control solution:**
An appropriate amount of a drug substance (control, 10 mg) was precisely weighed and added to a 10-mL brown measuring flask. Water was added for dissolution, and the volume was brought to the mark. The solution was shaken homogeneously to give a 1 mg/mL control solution.
**4.2. K-R test solution (pH 7.4)**
0.37 g of $CaCl_2$ and 1.4 g of glucose were weighed and dissolved in an appropriate amount of water. 7.8 g of NaCl, 0.35 g of KCl, 1.37 g of $NaHCO_3$, 0.32 g of $NaH_2PO_4$, and 0.02 g of $MgCl_2$ were dissolved in an appropriate amount of water. The two solutions were slowly mixed homogeneously with stirring. The pH was adjusted to 7.4, and the volume was brought to 1000 mL with water. The solution was mixed homogeneously to give a K-R test solution.
**4.3. Preparation of blank intestinal perfusion solution**
According to the test method for in situ circulating intestinal perfusion in rats, the K-R test solution was injected into the small intestines of the rats for 2 h of circulating perfusion to give a blank intestinal incubation solution.
**4.4. Intestinal perfusion solutions of EJM-001 and EJM-002**
Appropriate amounts of EJM-001 and EJM-002 were precisely weighed and separately dissolved in the K-R test solution. The solutions were then separately diluted to give a solution containing EJM-001 with a mass concentration of 0.3 mg/mL and a solution containing EJM-002 with a mass concentration of 0.3 mg/mL. The resulting solutions were separately mixed homogeneously to give an EJM-001 intestinal perfusion solution and an EJM-002 intestinal perfusion solution.

**5. Study on intestinal absorption of EJM-001 and EJM-002 in rats**
This study mainly investigated the intestinal absorption effects of EJM-001 and EJM-002 and explored the absorption mechanism.

**5.1. HPLC determination method conditions for EJM-001 and EJM-002 in intestinal perfusion solution**
Octadecylsilane-bonded silica gel was used as a filler [Phenomenex Luna C18(2), 4.6 mm × 100 mm, 3 μm, or equivalent chromatographic column]; a 20% acetonitrile/water solution was used as the mobile phase; the column temperature was 30 °C; the detection wavelength was 220 nm; the injection volume was 10 μL.
**5.2. Experimental method for circulating intestinal perfusion**

In the experiment, a constant flow pump was used to pump the intestinal perfusion solution into the intestines of the rats at a constant flow rate. The day before the experiment, the tubing of the constant flow pump was soaked with the intestinal perfusion solution to be tested to fully saturate the tubing and eliminate the adsorption effect of the tubing. On the day of the experiment, an appropriate amount of intestinal perfusion solution was prepared, added to a measuring cylinder, mixed homogeneously, and placed on a magnetic stirrer at a maintained temperature of 37±1 °C and a rotation speed of 120 rpm to ensure the homogeneity of the perfusion solution.
SD rats (220±20 g) were fasted for 12 h before the experiment (with free access to water) and anesthetized by intraperitoneal (ip) injection of a 20% urethane solution at a dose of 1.5 g/200 g. The rats were fixed after anesthesia. The

abdomen was dissected along the midline of the abdomen (about 3 cm). The intestinal segment to be examined was carefully separated and selected, and small incisions were made at both ends. The intestinal lumen was flushed with normal saline at (37±1) °C, and then the cannulas were inserted and ligated with sterile surgical thread (keeping the inlet and outlet cannulas at the same height to avoid overflow of the perfusion solution due to gravity). The wound was covered with gauze soaked in normal saline for moisture retention and illuminated with surgical lamp light to maintain body temperature.

Firstly, a certain amount of the test perfusion solution was circularly perfused at a flow rate of 0.25 mL/min for 15 min (no perfusion solution was collected), and the flow rate was kept unchanged. 2 mL of the test solution was quickly pipetted using a pipette at 0 h, 0.25 h, 0.5 h, 1 h, 1.5 h, 2 h, and 2.5 h, and an equal amount of the K-R test solution was added. The test solution was stored, and the volume before sampling was recorded until the experiment ended at 2.5 h (the perfusion solution was collected from 15 min to 150 min). The rats were sacrificed. 10 μL of the test solution at each time point was precisely pipetted into a high performance liquid chromatograph, and the determination was performed under the chromatographic conditions described in item 5.1. The peak area was recorded, the content (mg) and the cumulative content (mg) were calculated, and the In curve was fitted with time (x) and the cumulative content (mg) (y).

The test results of EJM-001 and EJM-002 are shown in FIG. 7 and FIG. 8, respectively. The experimental results show that the slope of the In fitting curve was close to zero, indicating that EJM-001 and EJM-002 were barely absorbed in the intestines of rats. The intestinal recovery rate of such compounds was greater than 99%, meaning that over 99% of the compounds were not absorbed into the blood circulatory system through the intestines. When the compounds were orally administered to mice at 5 mg/kg, the contents at all time points were below the lower limit of quantification (3 ng/mL), indicating extremely low bioavailability for such compounds.

**Test Example 2**

**1. Test name**

Activity experiment on hyperuricemia mouse model induced by potassium oxonate and hypoxanthine.

2. Test objective

According to the method for modeling the hyperuricemia mouse model, the effect of the test samples on hyperuricemia mice was studied.

3. Test plan

| | |
|---|---|
| Date of study initiation | Five days before administration (Day -5) |
| Date of animal arrival | Five days before administration (Day -5) |
| Date of animal grouping | Day 1 |
| Date of administration of drug to be tested | Day 8 |
| Date of test termination | Day 15 |

**4. Samples**

Sample 1: EJM-001 was freshly prepared to 2 mg/mL using 0.5% CMC-Na once daily prior to use.
Sample 2: EJM-002 was freshly prepared to 2 mg/mL using 0.5% CMC-Na once daily prior to use.
Sample 3: EJM-003 was freshly prepared to 2 mg/mL using 0.5% CMC-Na once daily prior to use.
Allopurinol: The standard was freshly prepared to 2 mg/mL using 0.5% CMC-Na once daily prior to use.
Benzbromarone: The standard was freshly prepared to 2 mg/mL using 0.5% CMC-Na once daily prior to use.
50 mg/mL potassium oxonate: 0.5 g of potassium oxonate was weighed, and 10 mL of 0.5% CMC-Na was added. Fresh preparation was performed once daily prior to use.
60 mg/mL hypoxanthine: 0.6 g of hypoxanthine was weighed, and 10 mL of 0.5% CMC-Na was added. Fresh preparation was performed once daily prior to use.

**5. Animals**

### 5.1. Strain and supplier

SPF-grade C57BL/6J mice, provided by Hunan Slac Jingda Laboratory Animal Co., Ltd. (production license No.: SCXK (Xiang) 2019-0004).

### 5.2. Age, weight, and gender

C57BL/6J mice: 4-6 weeks old, weighing 20±2 g, all male.

### 5.3. Number of animals

C57BL/6J mice: 80 mice.

### 5.4. Animal feeding

#### 5.4.1. Cage

The experimental animals were kept in a controlled environment with cage change once weekly.

#### 5.4.2. Feed

The qualified feed was added once daily, and the experimental animals had free access to food. If the free access to food was affected due to the experimental needs, it would be faithfully recorded.

#### 5.4.3. Drinking water

Tap water was aliquoted into water bottles for free access.

#### 5.4.4. Environmental control of animal feeding room

The animals were kept in a controlled environment with a temperature of 16-26 °C and a daily temperature difference of no more than 4 °C. The daily temperature and humidity of the animal room were read and recorded by a benchtop hygrothermograph. A 12-hour light/dark cycle was maintained, and the animal room was ventilated no less than 8 times/h.

## 6. Test design

### 6.1. Quarantine environment adaptation

Before the start of the test, the animals were acclimated in the test room for about 5 days, and cage-side observations were made once daily.

### 6.2. Grouping

| Group | Number of animals (animal) | Test substance and dose | Concentration | Administration volume (mL/kg) | Administration route |
|---|---|---|---|---|---|
| Blank control group | 10 | 0.5%CMC-Na | - | 10 | Intragastric administration |
| Model control group | 10 | Potassium oxonate | 50mg/mL | 5 | Intragastric administration |
| | | Hypoxanthine | 60mg/mL | 5 | |
| Sample group 1 | 10 | EJM-001 | 2mg/mL | 10 | Intragastric administration |

(continued)

| Group | Number of animals (animal) | Test substance and dose | Concentration | Administration volume (mL/kg) | Administration route |
|---|---|---|---|---|---|
| Sample group 2 | 10 | EJM-002 | 2mg/mL | 10 | Intragastric administration |
| Sample group 3 | 10 | EJM-003 | 2mg/mL | 10 | Intragastric administration |
| Allopurinol group | 10 | Allopurinol | 2mg/mL | 10 | Intragastric administration |
| Benzbromarone group | 10 | Benzbromarone | 2mg/mL | 10 | Intragastric administration |

On Day 1, the animals were randomly divided into 8 groups according to the body weight, including a blank control group, a model control group, a sample group 1 to a sample group 3, a positive control allopurinol group, and a positive control benzbromarone group, with 10 animals in each group.

### 6.3. Modeling

From Day 1 to Day 15, all animals except for the blank control group were modeled. 50 mg/mL potassium oxonate and 60 mg/mL hypoxanthine were mixed homogeneously at a ratio of 1:1 daily, and the mixture was then intragastrically administered at 10 mL/kg once daily for modeling.

### 6.4. Administration frequency and cycle

From Day 8 to Day 15, the administration was performed 1 h after modeling once daily for 7 consecutive days.

### 6.5. Selection reasons for animal species and number, as well as administration dose and route

Mice are commonly used animals for such tests, and the number of animals is selected based on the minimum number that meets the biological evaluation and statistical requirements.
Selection of administration dose: same as the positive control.
The intragastric administration of the test sample was selected in accordance with the route of exposure of the test sample in use.

### 6.6. Observation and examination

#### 6.6.1. Cage-side observations

During the test, the animals were observed daily for food consumption, behavior, state, hair, and mortality.

#### 6.6.2. Examination

The body weight was measured once weekly, and the administration dose was recalculated based on the body weight. After the test was completed, the data statistics were electronically transmitted to the client.

#### 6.6.3. Blood collection

On Day 15, 4 h after administration, the eyeballs were removed to collect the blood, and the blood was left to stand for about 30 min. Serum was separated at 3000 rpm, and the uric acid concentration was measured by the LC-MS method. The results are shown in FIG. 9.
The results show that such compounds act on intestinal epithelial cells to reduce the uric acid concentration in the blood by activating and enhancing the transportation of uric acid from the blood side to the intestinal lumen side of uric acid transporters. Meanwhile, due to their extremely low bioavailability, they are not absorbed into the blood system and thus have almost no toxic and side effects on other parts of the body.

**Test Example 3**

**1. Test name**

Effect of EJM001 on ABCG2 gene expression in Caco-2 cells

**2. Caco-2 cell culture**

Caco-2 cells were purchased from BeNa Culture Collection Co., Ltd. and seeded on a 6-well plate at $3 \times 10^5$ cells/well. Culture conditions: DMEM medium 89% + 10% FBS + 1% P/S.

**3. Test sample preparation**

An appropriate amount of EJM001 was precisely weighed and added to a 1.5-mL centrifuge tube, and DMSO was added for dissolution. A stock solution with a concentration of 100 mM was prepared, and an appropriate amount of the stock solution was taken and diluted with a complete medium to 30 μM, 10 μM, 6 μM, 1.2 μM, and 0.24 μM.

**4. Experimental method**

Determination of the effect of EJM001 on the expression level of the ABCG2/Bcrp1 gene under the acting time: The qPCR experiment included a Control group and an EJM001 group, with factor investigations on intervention duration and concentration of the compound.

1) Cell culture: Caco-2 cells were plated on a 6-well plate (3 × 105/well) and adhered to the wall overnight.
2) Compound interference: The concentrations of the compound were 30 μM, 10 μM, 6 μM, 1.2 μM, and 0.24 μM, respectively. The acting times of the compound were 12 h, 24 h, and 48 h, and the cells were collected at the corresponding time points.
3) Total RNA extraction and reverse transcription:

20 μL of reverse transcription system: RNA: 1000 ng; 5× PrimeScript RT Master mix: 4 μL; DEPC water: up to 20 μL.
Reverse transcription program: reverse transcription at 55 °C for 15 min; inactivation at 85 °C for 5 min.

4) Detection of the expression levels of ACTIN (internal reference gene) and ABCG2 genes by qPCR, with each sample loaded in triplicate for each gene.

20 μL of qPCR system: Primer mix (5 μM): 1.2 μL; cDNA: 8 μL; 2× mix (SYBER GREEN): 10 μL; DEPC water: 0.4 μL; ROX II: 0.4 μL.
qPCR reaction program: denaturation: 95 °C, 5 min, 1 cycle; annealing and extension: 95 °C, 10 s, 60 °C, 30 s, 95 °C, 15 s, 40 cycles; dissolution curve: 60 °C, 1 min, 95 °C, 15 s, 1 cycle.

**5. Data analysis**

1. Calculation formula for tumor cell growth inhibition rate:

Inhibition rate = [((OD540 control well - OD540 blank well) - (OD540 administration well - OD540 blank well))/(OD540 control well - OD540 blank well)] × 100%

The control wells were cell wells to which DMSO was added with a volume equal to that of the drug, the administration wells were cell wells to which a certain concentration of the drug was added, and the blank wells were wells containing only a medium.
2. Plotting of drug dose-response curves and calculation of half maximal inhibitory concentration (IC50):
According to the cell growth inhibition rates at each calculated drug concentration, the dose-response curve of the drug was plotted using GraphPad Prism8.0.1 software, and the IC50 value of the drug was calculated using the statistical method inherent in GraphPad Prism8.0.1 software.
3. Calculation formula for qPCR relative quantification:

$$\text{Fold gene expression} = 2\text{^}-(\Delta\Delta Ct) = 2\text{^}(\Delta(Ct(\text{control group}) - Ct(\text{drug treatment group})) \times 100000)$$

where $\Delta Ct$ = Ct (target gene) - Ct (internal reference gene), and the Ct value is the number of reaction cycles in which the fluorescence signal reaches the threshold in the PCR reaction, and is obtained by analysis with the instrument QuantStudio 5.

**6. Experimental results**

Intervention treatments of Caco2 cells were carried out with five different concentrations of EJM001 (0.24 μM, 1.2 μM, 6 μM, 10 μM, and 30 μM) at different times (see FIG. 10). Compared with the control at each time point, the EJM001 group with a concentration of 10 μM and the EJM001 group with a concentration of 30 μM showed significant upregulation for the ABCG2 gene in Caco2 cells, with significant differences (t test), and their P values were 0.0397 and <0.0001, respectively, while a low concentration of EJM001 had no significant effect on the ABCG2 gene in Caco2 cells. In conclusion, after Caco-2 cells were treated with 10 μM and 30 μM of EJM001 for 48 h, the transcription level of the ABCG2 gene in the Caco-2 cell model significantly increased.

The embodiments of the technical solutions of the present disclosure have been described above by way of example. It should be understood that the protection scope of the present disclosure is not limited to the embodiments described above. Any modification, equivalent replacement, improvement, and the like made by those skilled in the art without departing from the spirit and principle of the present disclosure shall fall within the protection scope of the claims of the present application.

## Claims

1. A compound represented by formula (I) and a racemate, a stereoisomer, a tautomer, an isotopically labeled compound, a nitrogen oxide, a solvate, a polymorph, a metabolite, an ester, a prodrug, or a pharmaceutically acceptable salt thereof:

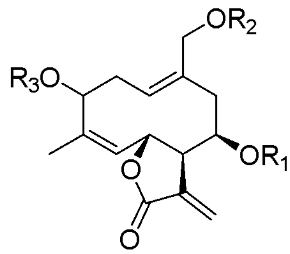

(I)

wherein

$R_1$ is selected from H, deuterium, and the following groups that are unsubstituted or optionally substituted with one, two, or more $R_a$: $C_{1-12}$ alkyl, $C_{3-12}$ cycloalkyl, 3- to 14-membered heterocyclyl, $C_{6-14}$ aryl, 5- to 14-membered heteroaryl, and -C(O)-$R_{11}$;

$R_2$ is selected from H, deuterium, and -C(O)-$R_{21}$ that is unsubstituted or optionally substituted with one, two, or more $R_b$;

$R_3$ is selected from H, deuterium, and $C_{1-12}$ alkyl, $C_{6-14}$ aryl, and 5- to 14-membered heteroaryl that are unsubstituted or optionally substituted with one, two, or more $R_c$;

$R_{11}$ and $R_{21}$ are identical or different and are independently selected from $C_{1-12}$ alkyl, $C_{2-12}$ alkenyl, $C_{2-12}$ alkynyl, $C_{3-12}$ cycloalkyl, 3- to 14-membered heterocyclyl, $C_{6-14}$ aryl, and 5- to 14-membered heteroaryl;

each of $R_a$, $R_b$, and $R_c$ is identical or different and is independently selected from H, deuterium, halogen, CN, OH, $C_{1-12}$ alkyl, and $C_{1-12}$ alkyloxy.

2. The compound and the racemate, the stereoisomer, the tautomer, the isotopically labeled compound, the nitrogen oxide, the solvate, the polymorph, the metabolite, the ester, the prodrug, or the pharmaceutically acceptable salt thereof according to claim 1, wherein $R_1$ is selected from H and $C_{1-6}$ alkyl, $C_{3-8}$ cycloalkyl, 3- to 8-membered heterocyclyl, $C_{6-10}$ aryl, 5- to 10-membered heteroaryl, and -C(O)-$R_{11}$ that are unsubstituted or optionally substituted with one, two, or more $R_a$;

preferably, $R_1$ is selected from H, methyl, ethyl, isopropyl, cyclopropyl, tetrahydropyranyl, piperidyl, phenyl, pyridinyl, pyrimidinyl, methylpyrrolyl, methylimidazolyl, thienyl, naphthyl, quinolyl, methylpyridinyl, tolyl, fluorophenyl, and

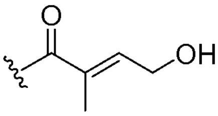
;

preferably, $R_1$ is selected from H, methyl, ethyl, isopropyl, cyclopropyl, phenyl,

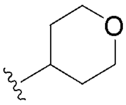 , 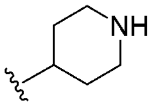 ,

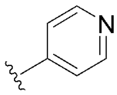 , 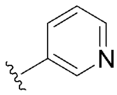 , 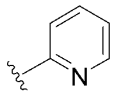 , 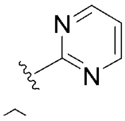 , 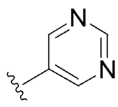 , 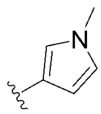 , 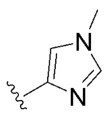 , 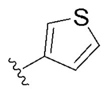 ,

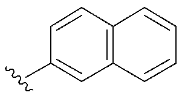 , 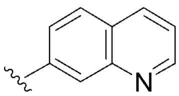 , 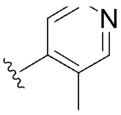 , 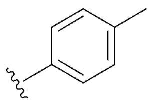 , 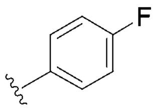 , and 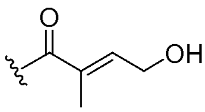 .

**3.** The compound and the racemate, the stereoisomer, the tautomer, the isotopically labeled compound, the nitrogen oxide, the solvate, the polymorph, the metabolite, the ester, the prodrug, or the pharmaceutically acceptable salt thereof according to claim 1 or 2, wherein $R_2$ is selected from H and -C(O)-$R_{21}$;
preferably, $R_2$ is H.

**4.** The compound and the racemate, the stereoisomer, the tautomer, the isotopically labeled compound, the nitrogen oxide, the solvate, the polymorph, the metabolite, the ester, the prodrug, or the pharmaceutically acceptable salt thereof according to any one of claims 1-3, wherein $R_3$ is selected from H, deuterium, and $C_{1-6}$ alkyl and $C_{6-10}$ aryl that are unsubstituted or optionally substituted with one, two, or more $R_c$.

**5.** The compound and the racemate, the stereoisomer, the tautomer, the isotopically labeled compound, the nitrogen oxide, the solvate, the polymorph, the metabolite, the ester, the prodrug, or the pharmaceutically acceptable salt thereof according to any one of claims 1-4, wherein $R_{11}$ is selected from the following groups that are unsubstituted or optionally substituted with one, two, or more $R_a$: $C_{1-6}$ alkyl, $C_{2-6}$ alkenyl, $C_{2-6}$ alkynyl, $C_{6-10}$ aryl, and 5- to 10-membered heteroaryl;

preferably, $R_{11}$ is selected from

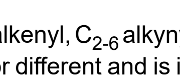
;

preferably, $R_{21}$ is selected from $C_{1-6}$ alkyl, $C_{2-6}$ alkenyl, $C_{2-6}$ alkynyl, $C_{6-10}$ aryl, and 5- to 10-membered heteroaryl;
preferably, each of $R_a$, $R_b$, and $R_c$ is identical or different and is independently selected from H, OH, F, and $C_{1-6}$ alkyl (e.g., methyl).

**6.** The compound and the racemate, the stereoisomer, the tautomer, the isotopically labeled compound, the nitrogen oxide, the solvate, the polymorph, the metabolite, the ester, the prodrug, or the pharmaceutically acceptable salt thereof according to any one of claims 1-5, wherein the compound of formula (I) has a structure represented by formula (I'):

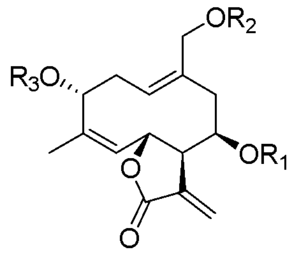

(I')

wherein $R_1$, $R_2$, and $R_3$ are independently defined as described in any one of claims 1-5.

**7.** The compound and the racemate, the stereoisomer, the tautomer, the isotopically labeled compound, the nitrogen oxide, the solvate, the polymorph, the metabolite, the ester, the prodrug, or the pharmaceutically acceptable salt thereof according to any one of claims 1-6, wherein the compound of formula (I) has a structure represented by formula (I-1) or (I-2):

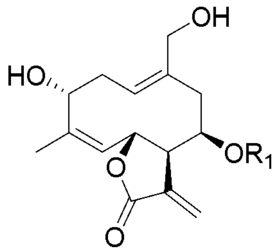

(I-1)

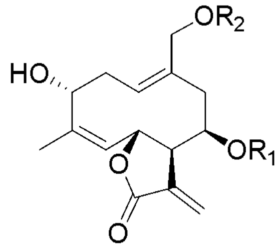

(I-2)

wherein $R_1$ and $R_2$ are independently defined as described in any one of claims 1-6; preferably, the compound of formula (I) has a structure represented by formula (II):

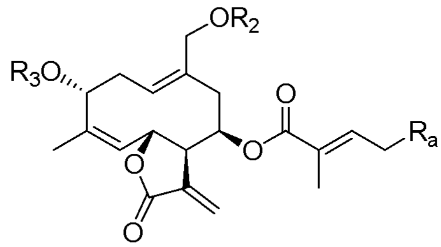

(II)

wherein $R_2$, $R_3$, and $R_a$ are independently defined as described in any one of claims 1-6; preferably, the compound of formula (I) has a structure represented by formula (II-1):

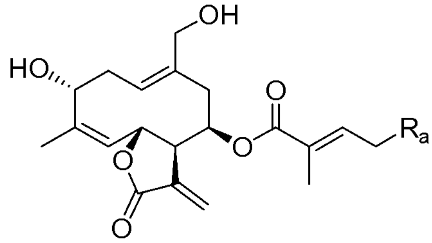

(II-1)

wherein $R_a$ is defined as described in any one of claims 1-6.

**8.** The compound and the racemate, the stereoisomer, the tautomer, the isotopically labeled compound, the nitrogen

oxide, the solvate, the polymorph, the metabolite, the ester, the prodrug, or the pharmaceutically acceptable salt thereof according to any one of claims 1-7, wherein the compound of formula (I) is selected from the following structures:

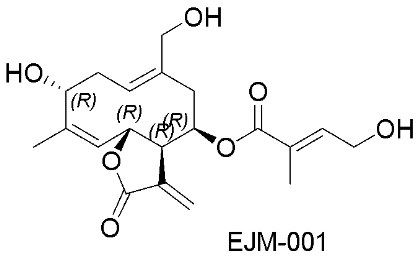
EJM-001 ,

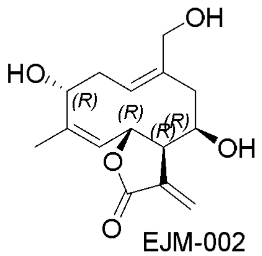
EJM-002 ,

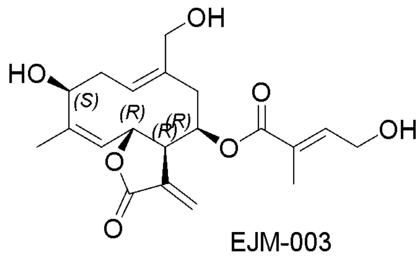
EJM-003 ,

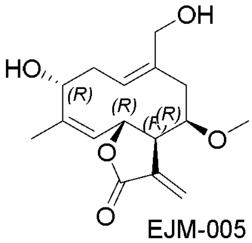
EJM-005 ,

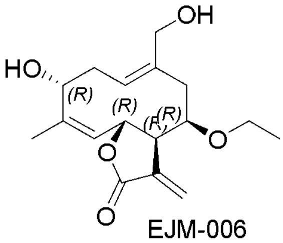
EJM-006 ,

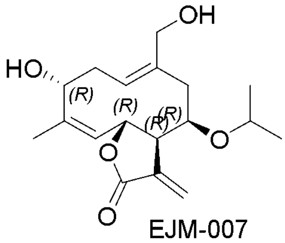
EJM-007 ,

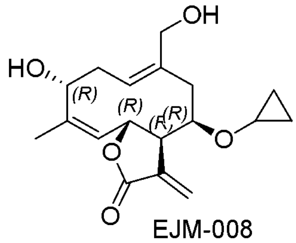
EJM-008 ,

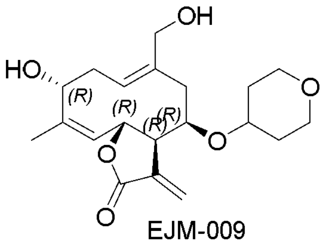
EJM-009 ,

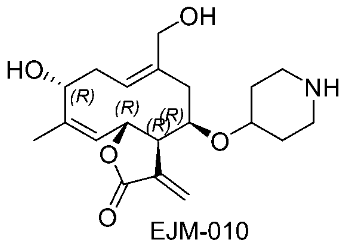
EJM-010 ,

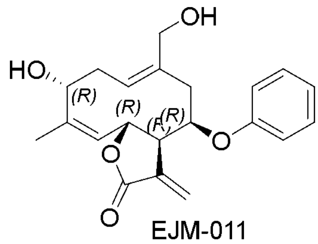
EJM-011 ,

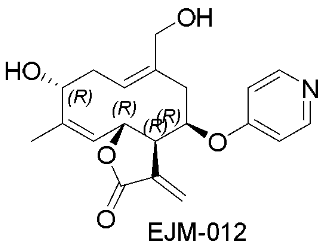
EJM-012 ,

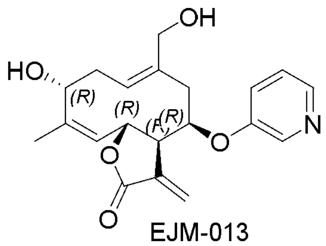
EJM-013 ,

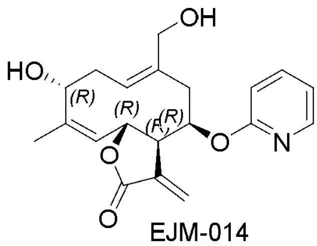
EJM-014 ,

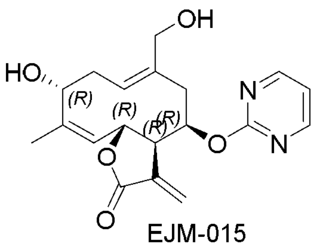
EJM-015 ,

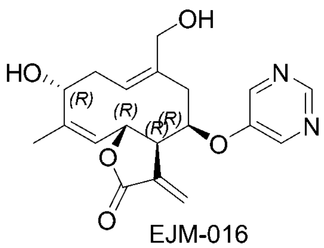
EJM-016 ,

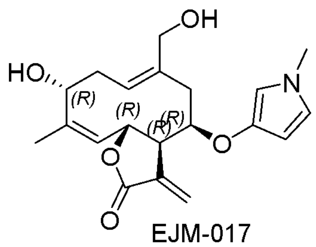
EJM-017 ,

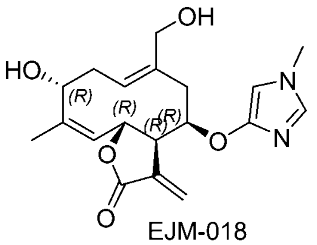
EJM-018 ,

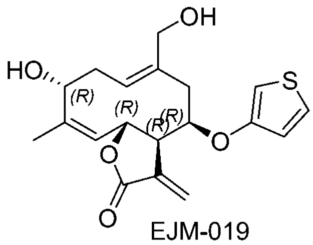
EJM-019 ,

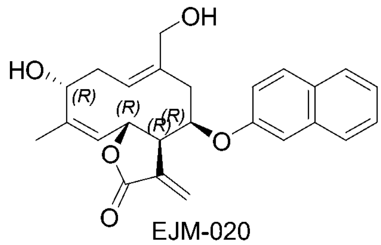
EJM-020 ,

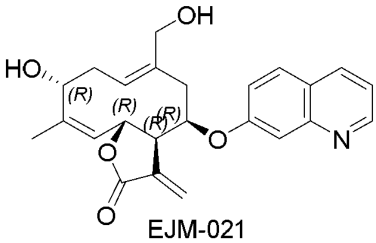
EJM-021 ,

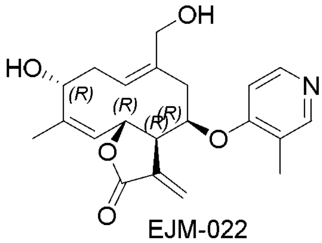
EJM-022 ,

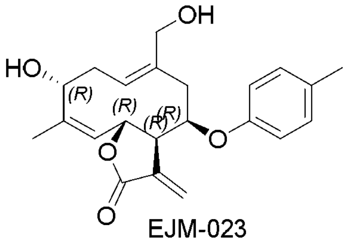
EJM-023 ,

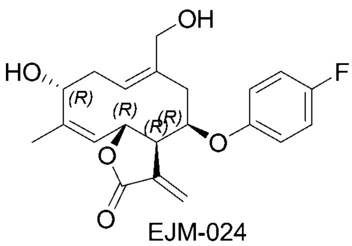
EJM-024 ,

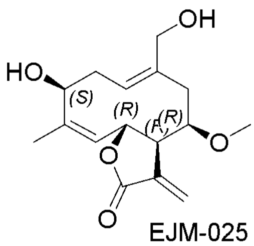
EJM-025 ,

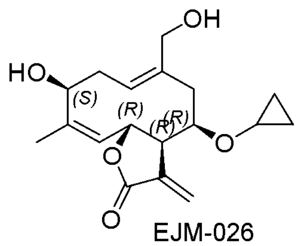
EJM-026 ,

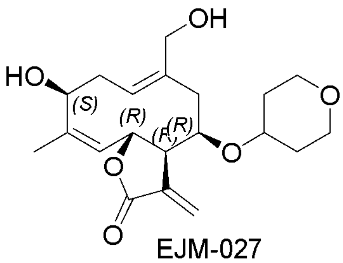
EJM-027 ,

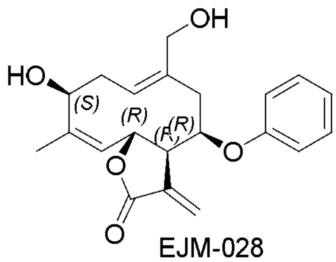
EJM-028 ,

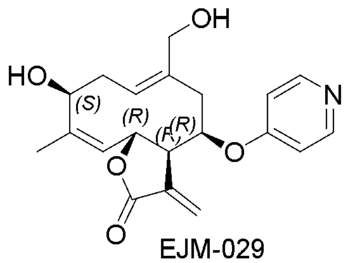
EJM-029 ,

and

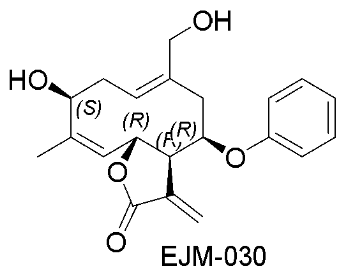
EJM-030 .

**9.** A pharmaceutical composition, comprising the compound and the racemate, the stereoisomer, the tautomer, the isotopically labeled compound, the nitrogen oxide, the solvate, the polymorph, the metabolite, the ester, the prodrug, or the pharmaceutically acceptable salt thereof according to any one of claims 1-8.

**10.** Use of the compound and the racemate, the stereoisomer, the tautomer, the isotopically labeled compound, the nitrogen oxide, the solvate, the polymorph, the metabolite, the ester, the prodrug, or the pharmaceutically acceptable salt thereof according to any one of claims 1-8, or the pharmaceutical composition according to claim 9 for the

manufacturing of a medicament, wherein

preferably, the medicament is a medicament for diagnosing, preventing, and/or treating a disease or condition caused by abnormal uric acid levels;
preferably, the medicament is a uric acid transporter agonist;
preferably, the disease or condition is selected from hyperuricemia and gout.

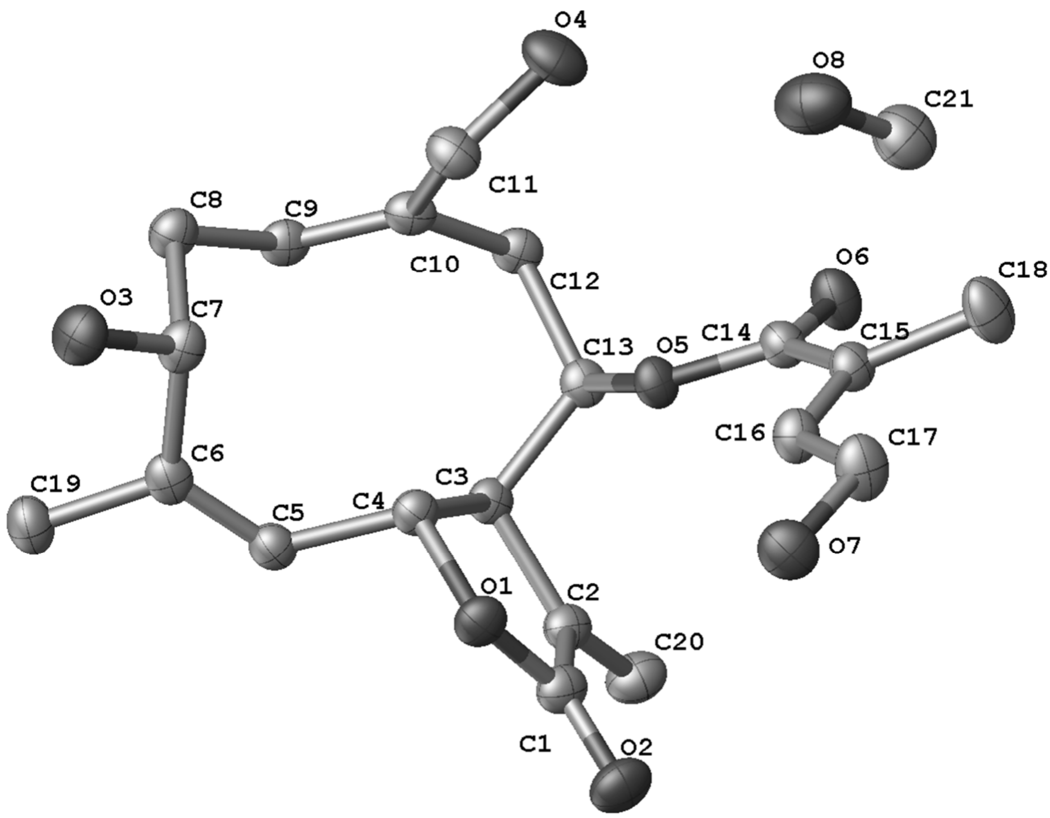

FIG. 1

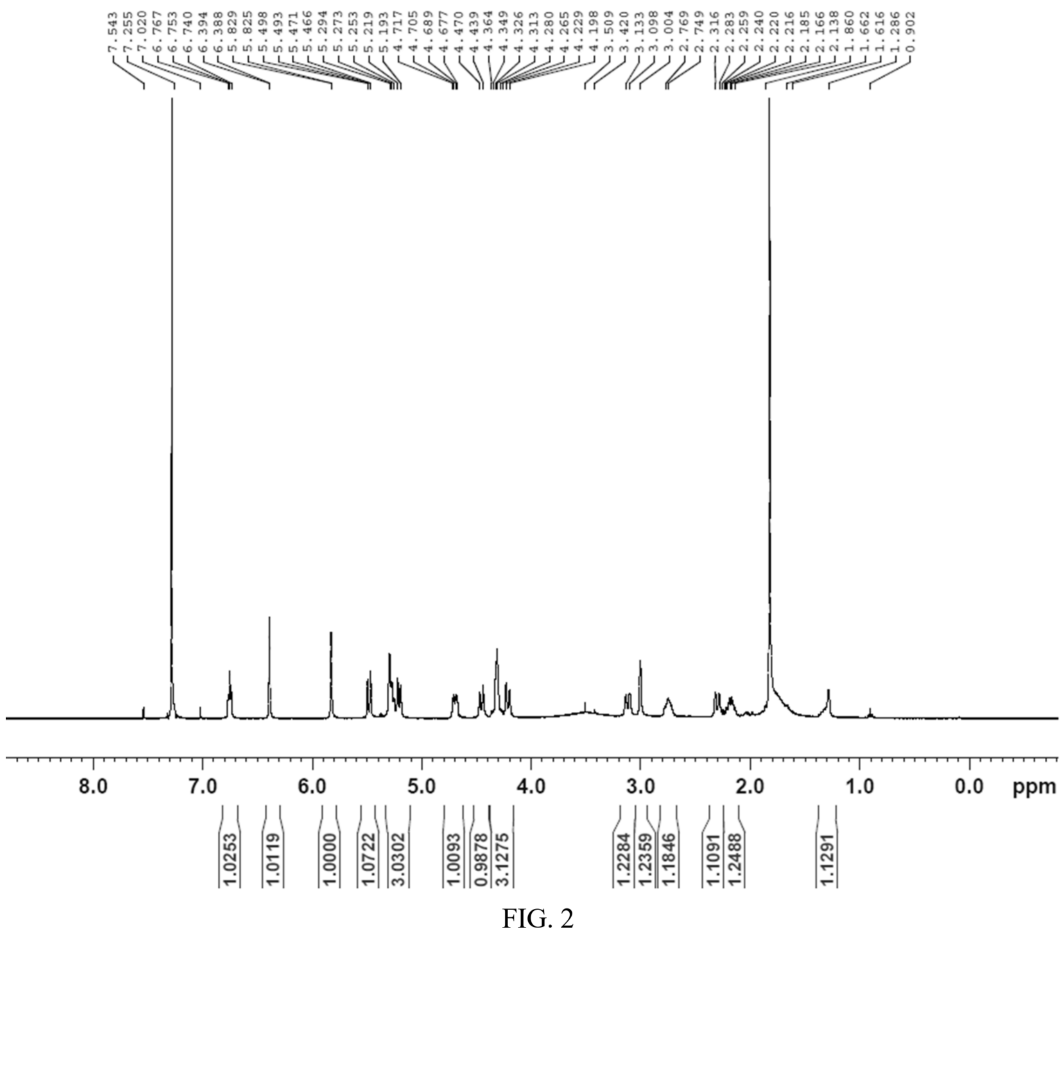
7.543
7.255
7.020
6.767
6.753
6.740
6.394
6.388
5.829
5.825
5.498
5.493
5.471
5.466
5.294
5.273
5.253
5.219
5.193
4.717
4.705
4.689
4.677
4.470
4.439
4.364
4.349
4.326
4.313
4.280
4.265
4.229
4.198
3.509
3.420
3.133
3.098
3.004
2.769
2.749
2.316
2.283
2.259
2.240
2.220
2.216
2.185
2.166
2.138
1.860
1.662
1.616
1.286
0.902
8.0
7.0
6.0
5.0
4.0
3.0
2.0
1.0
0.0
ppm
1.0253
1.0119
1.0000
1.0722
3.0302
1.0093
0.9878
3.1275
1.2284
1.2359
1.1846
1.1091
1.2488
1.1291

FIG. 2

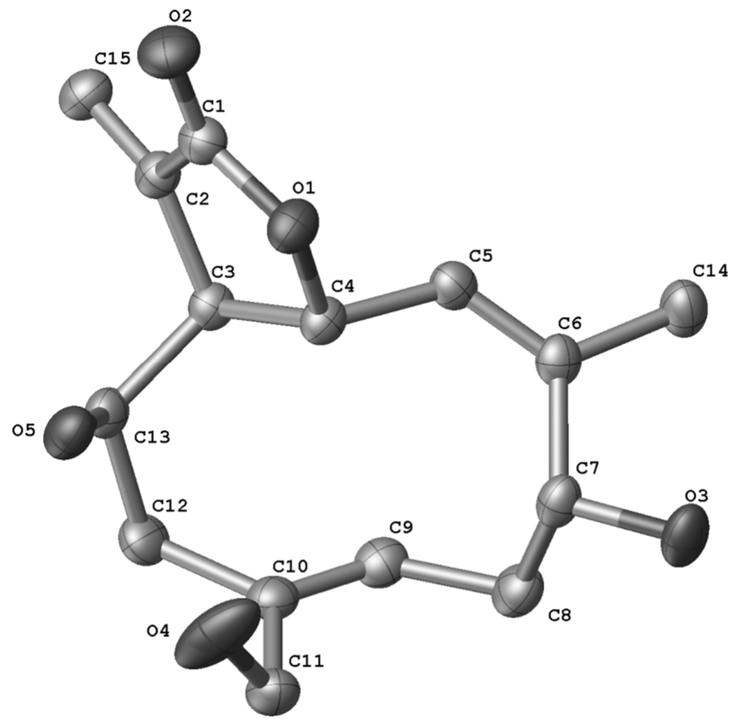

FIG. 3

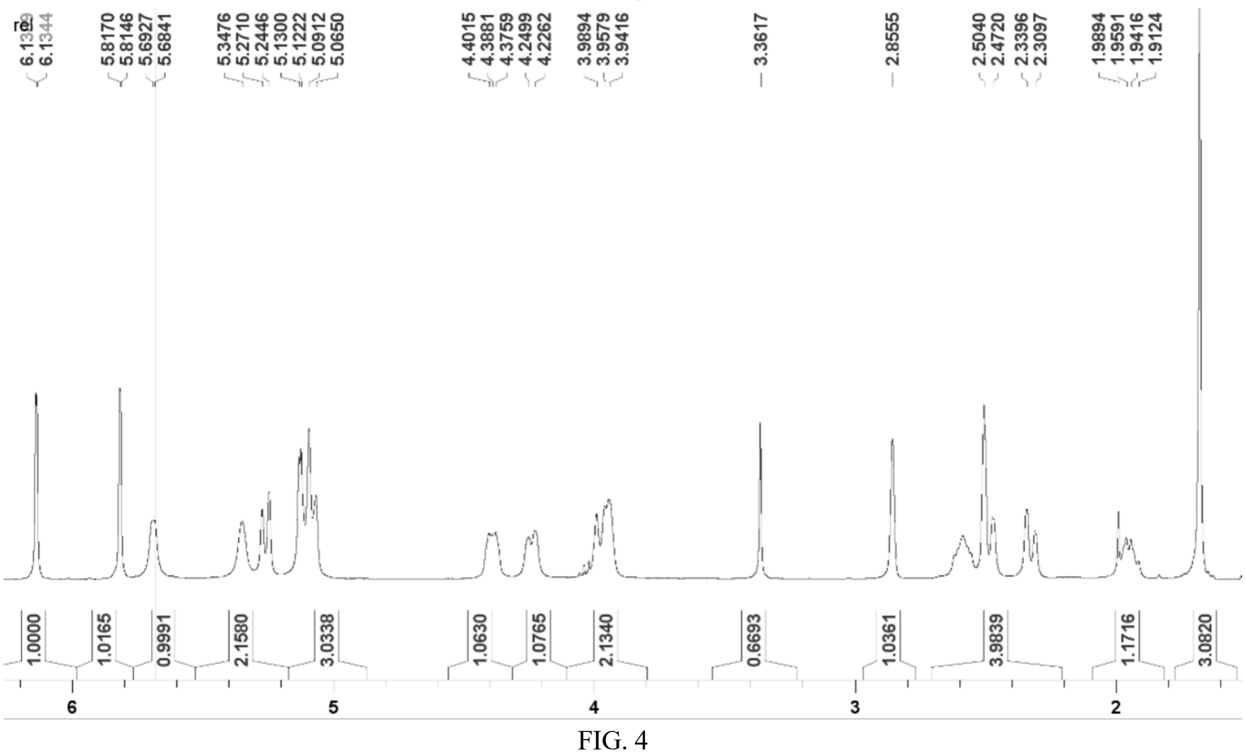
rel
6.1359
6.1344
5.8170
5.8146
5.6927
5.6841
5.3476
5.2710
5.2446
5.1300
5.1222
5.0912
5.0650
4.4015
4.3881
4.3759
4.2499
4.2262
3.9894
3.9579
3.9416
3.3617
2.8555
2.5040
2.4720
2.3396
2.3097
1.9894
1.9591
1.9416
1.9124
1.0000
1.0165
0.9991
2.1580
3.0338
1.0630
1.0765
2.1340
0.6693
1.0361
3.9839
1.1716
3.0820
6
5
4
3
2

FIG. 4

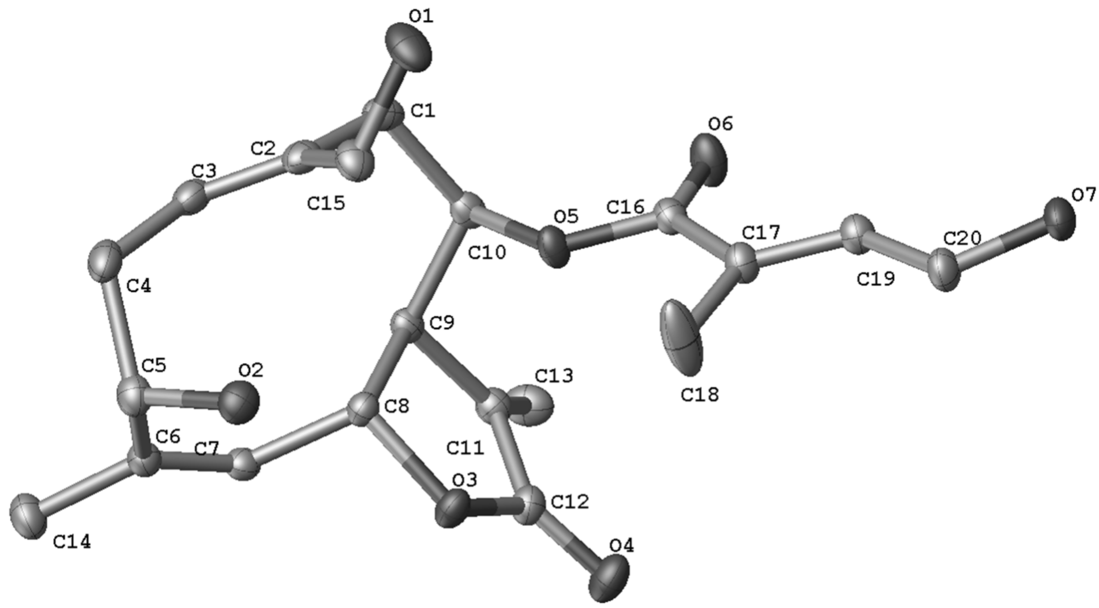

FIG. 5

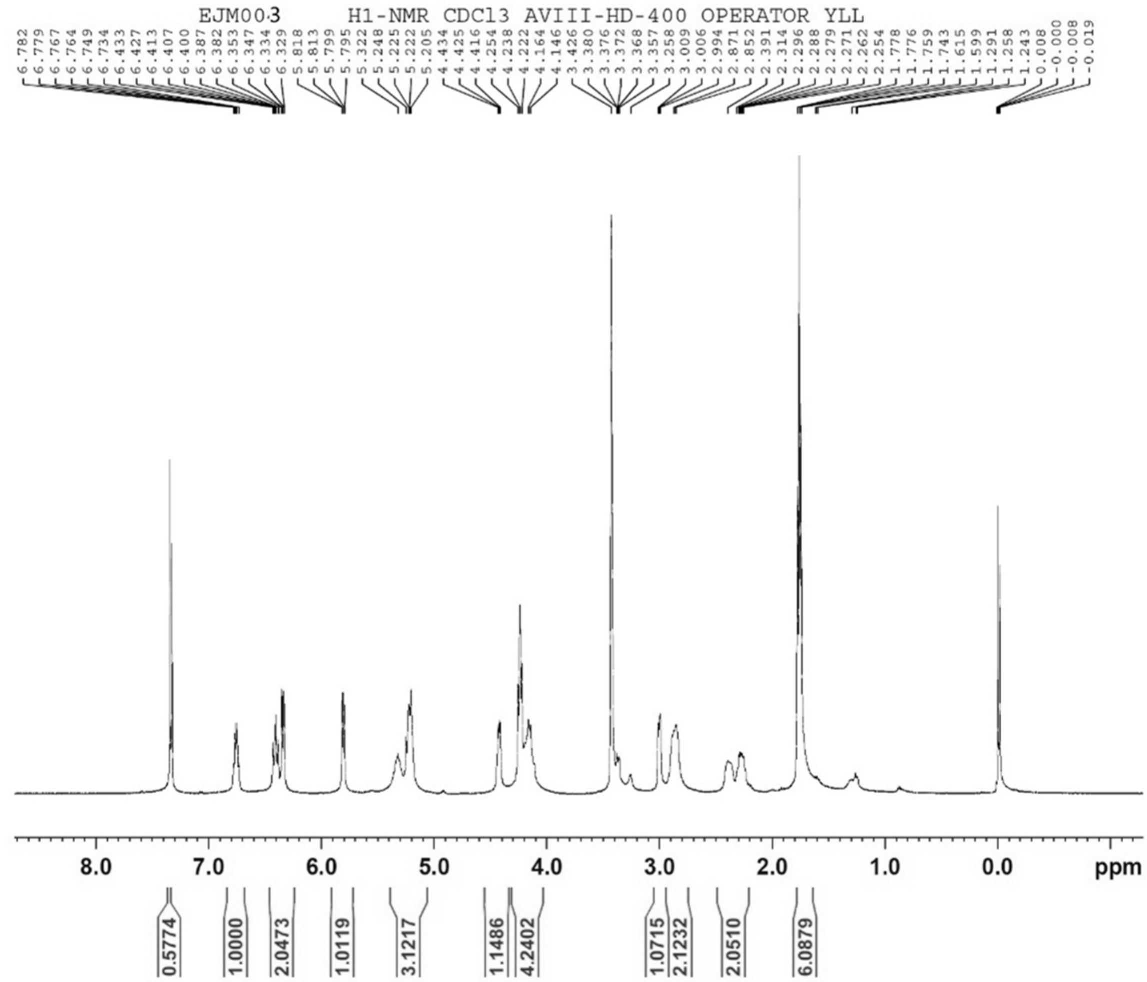
EJM003 H1-NMR CDCl3 AVIII-HD-400 OPERATOR YLL
6.782 6.779 6.767 6.764 6.749 6.734 6.433 6.427 6.413 6.407 6.400 6.387 6.382 6.353 6.347 6.334 6.329 5.818 5.813 5.799 5.795 5.322 5.248 5.225 5.222 5.205 4.434 4.425 4.416 4.254 4.238 4.222 4.164 4.146 3.426 3.380 3.376 3.372 3.368 3.357 3.258 3.009 3.006 2.994 2.871 2.852 2.391 2.314 2.296 2.288 2.279 2.271 2.262 2.254 1.776 1.759 1.743 1.615 1.599 1.291 1.258 1.243 0.008 -0.000 -0.008 -0.019
8.0 7.0 6.0 5.0 4.0 3.0 2.0 1.0 0.0 ppm
0.5774 1.0000 2.0473 1.0119 3.1217 1.1486 4.2402 1.0715 2.1232 2.0510 6.0879

FIG. 6

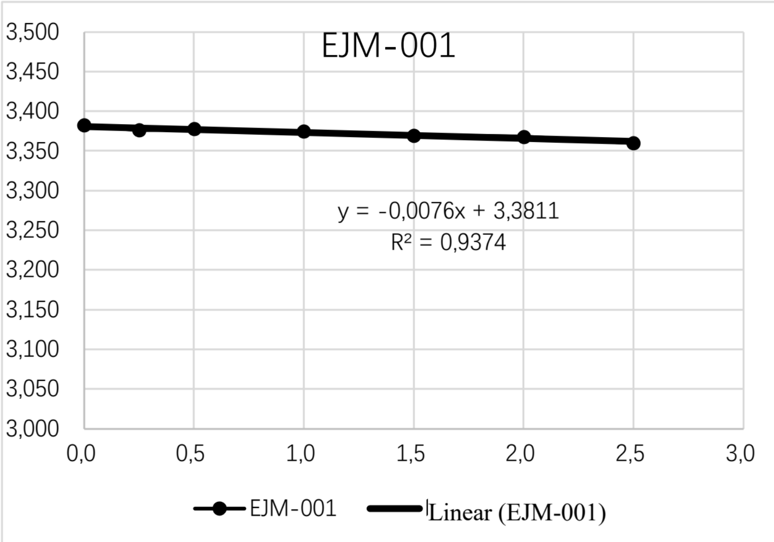


FIG. 7

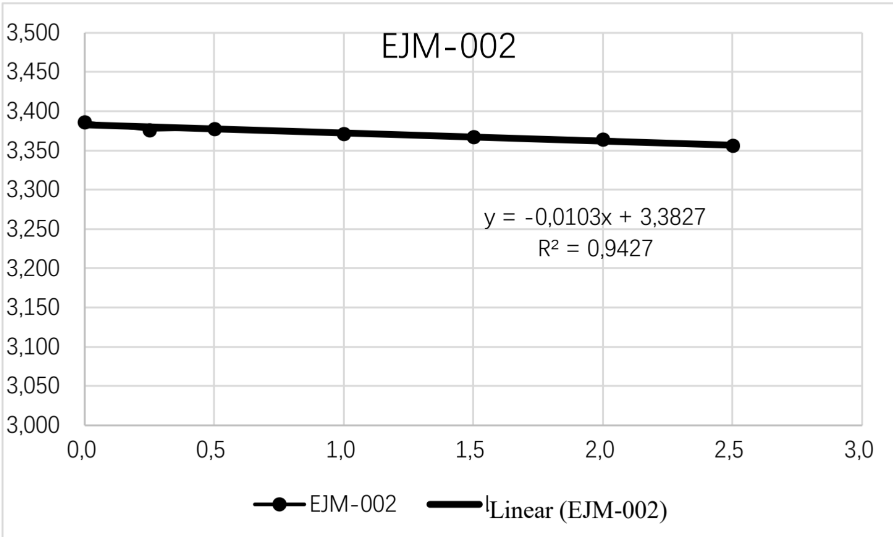


FIG. 8

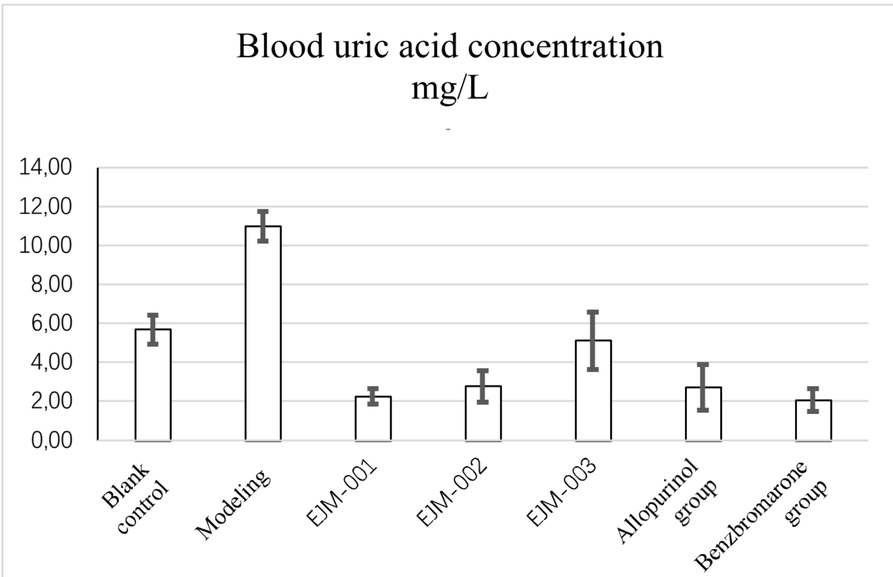


FIG. 9

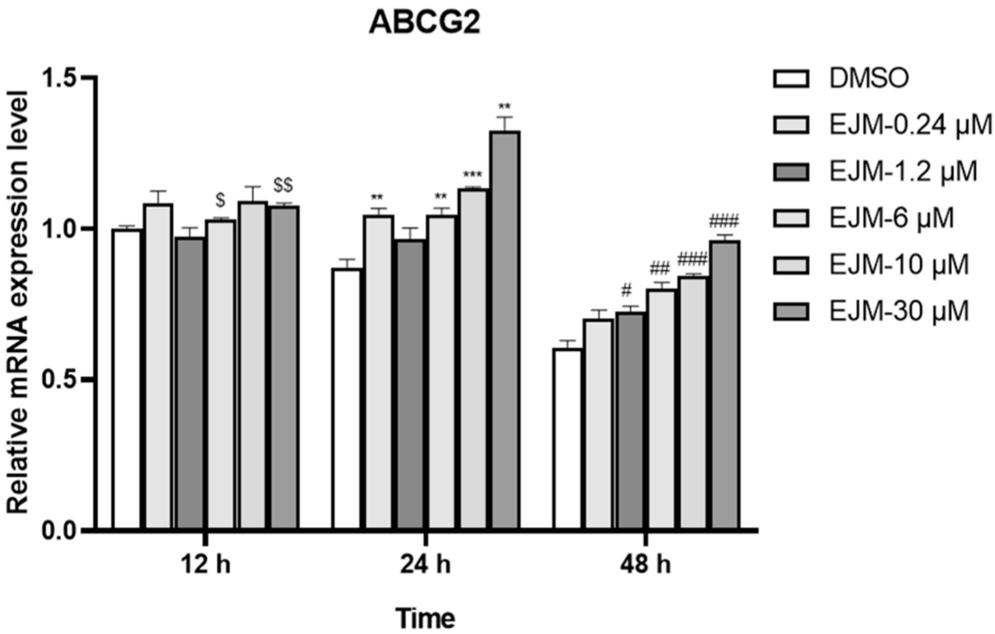
ABCG2
Relative mRNA expression level
1.5
1.0
0.5
0.0
$
$$
**
**
***
**
#
##
###
###
12 h
24 h
48 h
Time
DMSO
EJM-0.24 μM
EJM-1.2 μM
EJM-6 μM
EJM-10 μM
EJM-30 μM

FIG. 10

## INTERNATIONAL SEARCH REPORT

International application No. **PCT/CN2024/089520**

**A. CLASSIFICATION OF SUBJECT MATTER**

A61K31/365(2006.01)i; A61P13/12(2006.01)i; C07D307/93(2006.01)i; C07D493/00(2006.01)i

According to International Patent Classification (IPC) or to both national classification and IPC

**B. FIELDS SEARCHED**

Minimum documentation searched (classification system followed by classification symbols)

IPC: C07D, A61P, A61K, A23L

Documentation searched other than minimum documentation to the extent that such documents are included in the fields searched

Electronic data base consulted during the international search (name of data base and, where practicable, search terms used)

CNTXT, ENTXTC, CNKI, Registry (STN), CAplus (STN): 结构检索, 尿酸, 高尿酸血症, 痛风, 肾, uric acid, hyperuricemia, gout, kidney

**C. DOCUMENTS CONSIDERED TO BE RELEVANT**

| Category* | Citation of document, with indication, where appropriate, of the relevant passages | Relevant to claim No. |
|---|---|---|
| X | HERNÁNDEZ, L. R. et al. "Sesquiterpene Lactones from Stevia Vaga" *Phytochemistry,* Vol. 42, No. 5, 31 December 1996 (1996-12-31), pages 1369-1373 | 1-7 |
| X | Registry. "RN 756895-75-7 AND RN 756895-76-8" *STN,* 05 October 2004 (2004-10-05), pages 1-4 | 1-7 |
| PX | CN 116354912 A (HANGZHOU YIJIAN BIOMEDICAL TECHNOLOGY CO., LTD.) 30 June 2023 (2023-06-30) claims 2 and 4 | 1-7 |
| A | CN 105949156 A (LIU YU) 21 September 2016 (2016-09-21) claims 1 and 9 | 1-10 |
| A | CN 110638812 A (SUZHOU KAIXIANG BIOTECHNOLOGY CO., LTD.) 03 January 2020 (2020-01-03) claim 1, and abstract | 1-10 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 July 2024** | **31 July 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/089520**

| Patent document cited in search report | | | Publication date (day/month/year) | Patent family member(s) | Publication date (day/month/year) |
|---|---|---|---|---|---|
| CN | 116354912 | A | 30 June 2023 | None | |
| CN | 105949156 | A | 21 September 2016 | None | |
| CN | 110638812 | A | 03 January 2020 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2023104672331 A